# FASCICULE DE BREVET EUROPEEN

(11) **EP 0 673 926 B1**
(45) Date de publication et mention de la délivrance du brevet: **09.04.1997**
(21) Numéro de dépôt: 95400344.8
(22) Date de dépôt: 17.02.1995
(51) Int. Cl.: C07C 323/36, C07C 323/37, C07C 323/41, C07C 323/43, C07C 323/66, C07C 317/36, A61K 7/13

(54) **2-nitro p-phénylènediamines soufrées en position 5, leur procédé de préparation, compositions tinctoriales les contenant et leur utilisation en teinture des fibres kératiniques**
2-Nitro-p-Phenylendiamine, die in 5-Stellung ein Schwefelatom aufweisen, ihr Herstellungsverfahren, sie enthaltende Färbemittelzusammensetzungen und ihre Verwendung zum Färben von Keratinfasern
2-Nitro-p-phenylenediamines having a sulfur atom in position 5, process for their preparation, dye compositions containing them and their use for dyeing keratinic fibres

(30) Priorité: 24.03.1994 FR 9403451
(43) Date de publication de la demande: 27.09.1995
(73) Titulaire: L'OREAL, 75008 Paris (FR)
(72) Inventeur: Lagrange, Alain, F-77700 Coupvray (FR); Genet, Alain, F-93600 Aulnay-sous-Bois (FR)
(74) Mandataire: Andral, Christophe André Louis

(56) Documents cités:
- EP-A- 0 203 446
- EP-A- 0 303 826
- EP-A- 0 314 162
- FR-A- 2 692 572
- FR-A- 2 692 573

## Description

La présente invention concerne de nouveaux composés nitrés benzéniques du type nitro-paraphénylènediamines soufrées en position 5. Ces composés sont notamment destinés à la teinture des fibres kératiniques et en particulier des cheveux humains.

EP-A-303826 concerne des composés nitrés benzéniques du type 2-nitrométaphénylènediamines substituées.

Il existe principalement deux grands types de coloration capillaire. Le premier est la coloration semi-permanente ou coloration directe qui fait appelle à des colorants capables d'apporter à la coloration naturelle des cheveux, une modification plus ou moins marquée résistant à 4 ou 5 shampooings. Ces colorants sont appelés colorants directs et sont mis en oeuvre sans agent oxydant. Le deuxième est la coloration permanente ou coloration d'oxydation. Celle-ci est réalisée avec des précurseurs de colorants dits "d'oxydation" qui sont des composés incolores ou faiblement colorés qui une fois mélangés à des produits oxydants, au moment de l'emploi, peuvent donner naissance par un processus de condensation oxydative à des composés colorés et colorants.

Dans le domaine de la coloration capillaire, l'utilisation des colorants directs est très répandue car ils présentent certains avantages par rapport aux précurseurs de colorants d'oxydation et, notamment, une diminution des risques potentiels d'allergie et l'absence de sensibilisation du cheveu due au processus oxydatif.

Parmi les colorants directs les plus utilisés figurent les dérivés nitrés benzéniques qui, d'une part, présentent une forte affinité pour le cheveu et qui d'autre part, grâce à la variété des substituants possibles, permettent de couvrir une large gamme de nuances allant du jaune au bleu en passant par le rouge.

Toutefois, la formulation de ces colorants pose des problèmes du fait de leur résistance au lavage, qui n'est pas satisfaisante.

La demanderesse a donc recherché d'autres colorants nitrés benzéniques présentant une bonne solubilité dans l'eau, dans les mélanges eau/alcool et plus généralement dans les supports de teinture usuels et qui conduisent, sur les cheveux, à des teintures stables au lavage, et aussi à la lumière, aux intempéries, et à la transpiration.

C'est à la suite de ces recherches que la demanderesse a découvert de nouvelles 2-nitro p-phénylènediamine soufrées en position 5 ayant pour formule (I) : 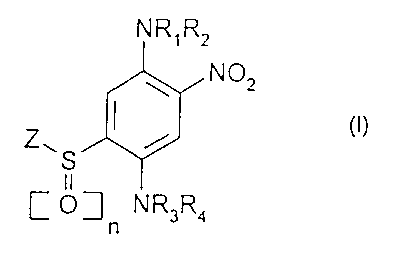 dans laquelle :
- n = 0, 1 ou 2 ;
- R₁, R₂, R₃, R₄ identiques ou différents représentent un atome d'hydrogène, un radical alkyke linéaire ou ramifié en C₁-C₄, monohydroxyalkyle en C₁-C₄, polyhydroxyalkyle en C₂-C₄, aminoalkyle en C₁-C₄, mono ou dialkyl(C₁-C₄)aminoalkyle en C₁-C₄
- Z représente un radical alkyle linéaire ou ramifié en C₁-C₄, monohydroxyalkyle en C₁-C₄, polyhydroxyalkyle en C₂-C₄, aminoalkyle en C₁-C₄, alcoxy(C₁-C₄)alkyle en C₁-C₄, acétylaminoalkyle en C₁-C₄, alkyl alkyl(C₁-C₄)aminoalkyle en C₁-C₄, dialkyl(C₁-C₄)aminoalkyle en C₁-C₄, trialkyl(C₁-C₄)ammonioalkyle en C₁-C₄ d'un halogénure d'alkyle en C₁-C₄ ou d'un alkylsulfate en C₁-C₄, benzyle éventuellement substitué, phényle éventuellement substitué, sulfoalkyle en C₁-C₄, alkoxy alkoxy(C₁-C₄)carbonylalkyle en C₁-C₄,

Les radicaux alkyle, hydroxyalkyle et alcoxyalkyle désignent notamment les radicaux méthyle, éthyle, propyle, butyle, hydroxyéthyle, hydroxypropyle, dihydroxypropyle, éthoxyéthyle.

Pour des raisons d'encombrement stérique, R₁ désigne de préférence un atome d'hydrogène.

Les composés de formule (I) peuvent être utilisés sous forme de base libre ou salifiée par des acides tels que l'acide chlorhydrique, l'acide bromhydrique, l'acide sulfurique etc. Ils peuvent donc se trouver sous forme de chlorhydrate, bromhydrate, sulfate, etc.

La présente invention a donc pour objet les nouveaux composés de formule (I) ainsi que leurs sels cosmétiquement acceptables.

Les composés de formule (I) préférés sont notamment choisis parmi les composés suivants :
- la 2-nitro 5-triméthylammonioéthylthio paraphénylènediamine,
- la 2-nitro 4-N-(β-hydroxyéthyl)amino 5-[(diméthyl)aminoéthyl]thio-aniline
- la 2-nitro 5-(β-aminoéthyl)thio paraphénylènediamine,
- la 2-nitro 4-N-(β,γ-dihydroxypropyl)amino 5-méthylthio aniline,
- la 2-nitro 5-méthylthio paraphénylènediamine,
- la 2-nitro 5-éthylthio paraphénylènediamine,
- la 2-nitro 5-butylthio paraphénylènediamine,
- la 2-nitro 5-β-hydroxyéthylthio paraphénylénediamine,
- la 2-nitro 5-(β,γ-dihydroxypropyl)thio paraphénylénediamine,
- le 1,4-N-di-(β-hydroxyéthyl)amino 2-nitro 5-éthoxyéthylthio benzène,
- la 2-nitro 4-N-(β-hydroxyéthylthio )amino 5-β-hydroxyéthylthio aniline,
- la 2-nitro 5-(paraméthoxybenzyl)thio paraphénylénediamine,
- la 2-nitro 4-N-(β-aminoéthyl)amino 5-méthylthio aniline,
- la 2-nitro 4-N-(diéthylaminoéthyl)amino 5-méthylthio aniline,
- la 2-nitro 4-N-(diéthylaminoéthyl)amino 5-éthylthio aniline,
- la 2-nitro 4-N-(β,γ-dihydroxypropyl)amino 5-acétylaminoéthylthio aniline,
- la 2-nitro 5-acétylaminoéthylthio paraphénylénediamine,
- la 2-nitro 5-[(diméthyl)aminoéthyl]thio paraphénylénediamine,
- la 2-nitro 5-(β-aminoéthyl)thio paraphénylénediamine,
- la 2-nitro 4-amino 5-(β-hydroxypropyl)thio )thio N-(méthyl)aniline,
- la 2-nitro 4-amino 5-(para-hydroxyphényl)thio N-(méthyl) aniline,
- la 2-nitro 5-éthoxycarbonyléthylthio paraphénylènediamine,
- la 2-nitro 5-méthylsulfinyl paraphénylénediamine,
- la 2-nitro 5-mésyl paraphénylénediamine,
- le 1-N-(β-hydroxyéthyl)amino 2-nitro 4,N,N-bis-(β-hydroxyéthyl)amino 5-isopropylthiobenzène,
- la 2-nitro 5-sulfoéthylthio paraphénylénediamine,
ainsi que leurs sels.

Parmi les composés de formule (I) plus particulièrement préférés, on peut citer :
- la 2-nitro 5-triméthylammonioéthylthio paraphénylénediamine,
- la 2-nitro 4-N-(β-hydroxyéthyl)amino-5-[(diméthyl)aminoéthyl]thioaniline,
- la 2-nitro 5-(β-aminoéthyl)thio paraphénylénediamine,
- la 2-nitro 4-N-(β,γ-dihydroxypropyl)amino )amino 5-méthylthio aniline,
ainsi que leur sels.

La présente invention a également pour objet un procédé de préparation des composés de formule (I).

Selon ce procédé, on fait réagir un composé de formule II 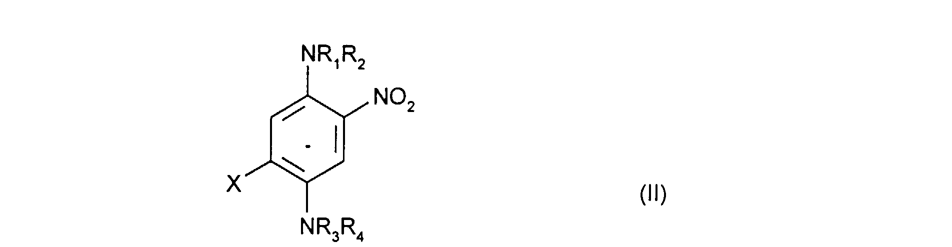 dans laquelle R₁, R₂, R₃ et R₄ ont les significations indiquées pour les composés de formule (I) ci-dessus et X représente un atome d'halogène choisi parmi le chlore, le brome et l'iode, avec un thiol de formule (III)

ASZ (III)

dans laquelle Z a les mêmes significations que celles indiquées ci-dessus dans la formule (I) et A est un atome d'hydrogène, de sodium ou de potassium, pour obtenir un composé de formule (I) dans lequel n = 0

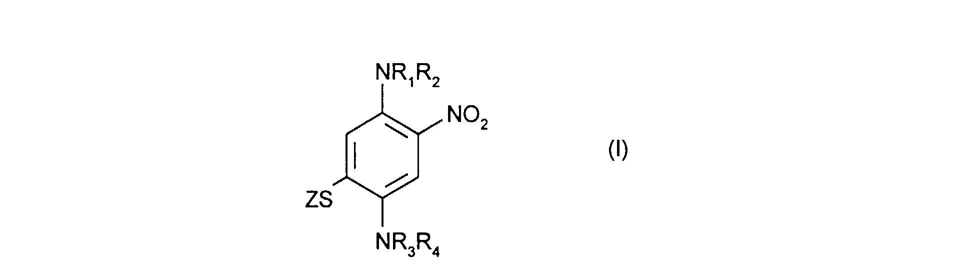

La substitution du groupement halogéno X est effectuée dans un solvant tel que le méthoxy 1,2-éthane, le diméthylformamide, la N-méthylpyrrolidone ou le dioxane. La température de la réaction est comprise entre la température ambiante et la température de reflux du milieu réactionnel. Les "capteurs" de l'acide halohydrique utilisés sont choisis de préférence parmi la soude, la potasse, la triéthylamine, les carbonates de sodium, de potassium et de calcium ainsi que les sels de sodium des thiols utilisés.

Les composés dans lesquels n est différent de zéro subissent ensuite, dans une deuxième étape, une oxydation adaptée à la valeur de n que l'on souhaite obtenir Cette oxydation est réalisée en milieu solvant en présence d'un agent oxydant classique du soufre tel que les persulfates. On peut citer à titre d'exemple le monopersulfate de potassium. Cette oxydation est de préférence réalisée à une température comprise entre zéro et 40 °C environ. Les composés de formule (I) ci-dessus sont de préférence préparés à la pression atmosphérique mais ils peuvent également être préparés sous pression.

Les composés de formule (I) ci-dessus peuvent être utilisés comme colorants et en particulier comme colorants directs pour la teinture des fibres kératiniques, et notamment des cheveux humains. Ces composés confèrent aux cheveux une coloration à dominante rouge.

Un autre objet de l'invention est donc une composition tinctoriale pour la coloration directe des fibres kératiniques, en particulier des cheveux humains, contenant dans un véhicule aqueux, alcoolique ou hydroalcoolique, au moins un composé de formule (I) indiquée ci-dessus ou l'un de leurs sels cosmétiquement acceptables.

Les composés de l'invention sont particulièrement appréciés pour obtenir des teintes variées en association avec d'autres colorants directs et notamment avec les colorants nitrés benzéniques classiques.

Selon un mode de réalisation préférentiel, la composition tinctoriale selon l'invention contient un composé de formule (I) ou l'un de ses sels cosmétiquement acceptables, en association avec un ou plusieurs colorants nitrés benzéniques jaune ou jaune vert donnant sur cheveux gris à 90 % de blancs une nuance ou "hue" comprise entre 2,4 Y et 0,2 YR sur le cercle de MUNSELL (voir publication de Official Digest, Avril 1975, page 375, figure 2).

La demanderesse a constaté que lorsqu'on utilise le colorant de formule (I), en association avec un ou plusieurs colorants classiques jaune ou jaune vert, on obtient, notamment sur des cheveux naturels gris à 90 % de blancs ou sur des cheveux gris permanentés, des nuances naturelles plus solides au lavage, à la lumière, aux intempéries et à la transpiration que lorsqu'on utilise les colorants de l'art antérieur.

Selon un mode de réalisation plus particulièrement préféré de la présente invention, le composé de formule (I) est associé aux colorants jaune ou jaune vert choisis parmi les composés suivants :
- le 1-β-hydroxyéthyloxy-3-méthylamino-4-nitrobenzène,
- le 1-(méthylamino)-2-nitro-5-(β, γ-dihydroxypropyl)oxybenzène,
- le 1-(β-hydroxyéthylamino)-2-méthoxy-4-nitrobenzène,
- le 1-(β-aminoéthylamino)-2-nitro-5-méthoxy-benzène,
- le 1,3-di(β-hydroxyéthylamino)-4-nitro-6-chlorobenzène,
- le 1-amino-2-nitro-6-méthyl-benzène,
- le 1-(β-hydroxyéthylamino)-2-hydroxy-4-nitrobenzène,
- la N-(β-hydroxyéthyl)-2-nitro-4-trifluorométhylaniline,
- l'acide 4-β-hydroxyéthylamino-3-nitro-benzènesulfonique,
- l'acide 4-éthylamino-3-nitro-benzoïque,
- le 4-(β-hydroxyéthyl)amino-3-nitro-chlorobenzène,
- le 4-(β-hydroxyéthyl)amino-3-nitro-méthylbenzène,
- le 4-(β,γ-dihydroxypropyl)amino-3-nitro-trifluorométhylbenzène,
- le 1-β-uréidoéthylamino-4-nitrobenzène,
- l'O,N-bis(β-hydroxyéthyl)-2-amino-5-nitrophénol,
- le 1,3-diamino-4-nitrobenzène,
- le 1-hydroxy-2-amino-5-nitrobenzène,
- le 1-amino-2-[tris(hydroxyméthyl)méthyl]amino-5-nitro-benzène,
- le 1-(β-hydroxyéthyl)amino-2-nitrobenzène,
- le 4-(β-hydroxyéthylamino)-3-nitrobenzamide.

Les composés de formule (I) selon l'invention peuvent également être associés à des colorants nitrés benzéniques bleus tels que par exemple :
- le 1-(β-hydroxyéthyl)amino-4-N,N-bis(β-hydroxyéthyl)amino 2-nitrobenzène,
- le 1-(γ-hydroxypropyl)amino 4-N,N-bis(β-hydroxyéthyl)amino 2-nitrobenzène,
- le 1-(β-hydroxyéthyl)amino-4-(N-méthyl, N-β-hydroxyéthyl)amino 2-nitrobenzène,
- le 1-(β-hydroxyéthyl)amino-4-(N-éthyl, N,β-hydroxyéthyl)amino 2-nitrobenzène,
- le 1-(β, γ-dihydroxypropyl)amino 4-(N-éthyl, N,β-hydroxyéthyl)amino 2-nitrobenzène,
- les 2-nitroparaphénylènediamines de formule : 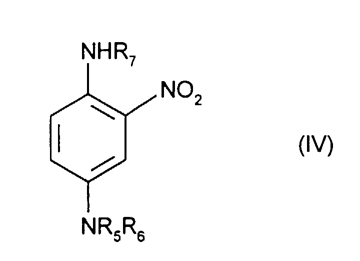
dans laquelle :
- R₅ représente un radical alkyle en C₁-C₄, β-hydroxyéthyle, β-hydroxypropyle ou γ-hydroxypropyle ;
- R₆ et R₇, indépendamment l'un de l'autre, représentent un radical β-hydroxyéthyle, β-hydroxypropyle, γ-hydroxypropyle, ou β,γ-dihydroxypropyle, l'un au moins des radicaux R₅, R₆ ou R₇ représentant un radical γ-hydroxypropyle et R₅ et R₆ ne pouvant désigner simultanément un radical β-hydroxyéthyle lorsque R₇ est un radical γ-hydroxypropyle, tels que décrits dans la demande de brevet FR 92-07515.

La concentration en composé de formule (I) est de préférence comprise entre 0,01 et 10 % en poids, et encore plus préférentiellement entre 0,1 et 5% en poids, exprimée en base libre, par rapport au poids total de la composition tinctoriale.

La concentration totale en colorants jaune et/ou jaune vert et/ou bleu est de préférence comprise entre 0,05 et 3 % en poids environ, sur la base du poids total de la composition tinctoriale.

On peut bien entendu ajouter aux associations de composés de formule (I) et de colorants jaune ou jaune vert ou bleu selon l'invention, d'autres colorants nitrés benzéniques, par exemple des colorants rouges choisis parmi les composés suivants :
- le 1-hydroxy-3-nitro-4-(γ-hydroxypropylamino)benzène,
- le N-(β-hydroxyéthyl)amino-3-nitro-4-aminobenzène,
- le 1-amino-3-méthyl-4-(β-hydroxyéthyl)amino-6-nitrobenzène,
- le 1-hydroxy-3-nitro-4-N-β-hydroxyéthyl aminobenzène,
- le 1,4-diamino-2-nitrobenzène,
- le 1-amino-2-nitro-4-méthylaminobenzène,
- la N-(β-hydroxyéthyl)-2-nitro-paraphénylènediamine,
- le 1-amino-2-nitro-4-(β-hydroxyéthylamino)-5-chlorobenzène,
- la 2-nitro-4-amino-diphénylamine,
- le 1-amino-3-nitro-6-hydroxybenzène.

On peut également ajouter des colorants nitrés benzéniques orangés choisis parmi les composés suivants :
- le 1-(β-aminoéthyl)amino-2-nitro-4-(β-hydroxyéthyl)oxybenzène,
- le 1-(β, γ-dihydroxypropyl)oxy-3-nitro-4-(β-hydroxyéthyl)aminobenzène,
- le 1-hydroxy-3-nitro-4-aminobenzène,
- le 1-hydroxy-2-amino-4,6-dinitrobenzène,
- le 1-méthoxy-3-nitro-4-(β-hydroxyéthylamino)benzène,
- la 2-nitro-4'-hydroxydiphénylamine,
- le 1-amino-2-nitro-4-hydroxy-5-méthylbenzène.

On peut aussi ajouter d'autres colorants directs tels que des colorants azoïques, des colorants anthraquinoniques, des colorants dérivés du triarylméthane ou des colorants basiques parmi lesquels on peut citer plus particulièrement les colorants connus sous les dénominations "Basic Brown 16", "Basic Yellow 57", "Basic Red 76" et "Basic Blue 99" dans le COLOR INDEX, 3ème édition.

La proportion de ces colorants d'addition, nitrés benzéniques rouges ou orangés ou autres colorants directs, peut varier entre 0,05 et 10 % en poids environ par rapport au poids total de la composition tinctoriale.

Les composés de formule (I) peuvent également être incorporés dans des compositions tinctoriales contenant des colorants d'oxydation tels que des bases d'oxydation et des coupleurs de façon à enrichir en reflets les nuances obtenues avec ces colorants d'oxydation.

La composition tinctoriale selon l'invention peut comprendre, comme véhicule approprié, l'eau et/ou des solvants organiques acceptables sur le plan cosmétique et plus particulièrement des alcools tels que l'alcool éthylique, l'alcool isopropylique, l'alcool benzylique et l'alcool phényléthylique ou des glycols ou éthers de glycols tels que, par exemple, l'éthylèneglycol et ses éthers monométhylique, monoéthylique et monobutylique, le propylèneglycol, le butylèneglycol, le dipropylèneglycol ainsi que les alkyléthers de diéthylèneglycol comme par exemple le monoéthyléther ou le mono-butyléther du diéthylèneglycol, dans des concentrations comprises entre 0,5 et 20 % et, de préférence, entre 2 et 10 % par rapport au poids total de la composition.

On peut également ajouter à la composition selon l'invention des amides gras tels que les mono- et diéthanolamides des acides dérivés du coprah, de l'acide laurique ou de l'acide oléïque, à des concentrations comprises entre 0,05 et 10 % en poids environ.

On peut encore ajouter à la composition selon l'invention des agents tensio-actifs anioniques, cationiques, non-ioniques, amphotères, zwittérioniques ou leurs mélanges. De préférence, les tensio-actifs sont présents dans la composition selon l'invention en une proportion comprise entre 0,1 et 50 % en poids environ et avantageusement entre 1 et 20 % en poids environ par rapport au poids total de la composition.

Parmi les agents tensio-actifs, on peut citer plus particulièrement les agents tensio-actifs anioniques utilisés seuls ou en mélange tels que, notamment, les sels alcalins, les sels de magnésium, les sels d'ammonium, les sels d'amine ou les sels d'alcanolamine des composés suivants :
- alkylsulfates, alkyléthersulfates, alkylamidesulfates éthoxylés ou non,
- alkylsulfonates, alkylamide sulfonates, alphaoléfinesulfonates ;
- alkylsulfoacétates, alkylphosphates ;
- acides gras tels que les acides laurique, myristique, oléïque, ricinoléïque, palmitique, stéarique, les acides d'huile de coprah ou d'huile de coprah hydrogénée, les acides carboxyliques d'éthers polyglycoliques, les radicaux alkyle de ces composés ayant une chaîne linéaire de 12 à 18 atomes de carbone.

A titre d'agents tensio-actifs cationiques, on peut citer plus particulièrement les sels d'amines grasses, les sels d'ammonium quaternaire tels que les chlorures et bromures d'alkyldiméthylbenzylammonium, d'alkyltriméthylammonium, d'alkyl-diméthylhydroxybenzylammonium, de diméthylalkylammonium, les sels d'alkylpyridinium, les dérivés d'imidazoline. Les groupements alkyle des dérivés d'ammonium quaternaire précités sont des groupements à chaîne longue ayant, de préférence, entre 12 et 18 atomes de carbone.

Parmi ces composés à caractère cationique on peut également citer les oxydes d'amines.

Parmi les agents tensio-actifs amphotères qui peuvent être utilisés, on peut citer en particulier les alkylamino (mono- et di) propionates, les bétaïnes telles que les alkyl-bétaïnes, les N-alkyl-sulfobétaïnes, les N-alkylaminobétaïnes, le radical alkyle ayant entre 8 et 22 atomes de carbone, les cycloimidiniums tels que les alkylimidazolines.

Parmi les tensio-actifs non ioniques qui peuvent éventuellement être utilisés dans les compositions conformes à l'invention, on peut mentionner les alcools, α-diols, alkylphénols et amides, polyglycérolés, ces composés comportant une chaîne grasse en C₈-C₁₈ ; les alcools, alkylphénols et acides gras, polyéthoxylés, ces composés comportant une chaîne grasse en C₈ à C₁₈ ; les condensats d'oxyde d'éthylène et de propylène sur des alcools gras ; les amides gras polyéthoxylés, contenant au moins 5 moles d'oxyde d'éthylène ; les amines grasses polyéthoxylées.

Les produits épaississants, que l'on peut ajouter dans la composition selon l'invention, peuvent avantageusement être pris dans le groupe formé par l'alginate de sodium, la gomme arabique, la gomme de guar, la gomme de caroube, la gomme de xanthane, les dérivés de la cellulose tels que la méthylcellulose, l'hydroxyéthylcellulose, l'hydroxypropylméthylcellulose, le sel de sodium de la carboxyméthylcellulose et les polymères d'acide acrylique.

On peut également utiliser des agents épaississants minéraux tels que la bentonite. Ces épaississants sont utilisés seuls ou en mélange et, de préférence, sont présents en une proportion comprise entre 0,2 et 5 % en poids environ par rapport au poids total de la composition et, avantageusement, entre 0,5 et 3 % en poids environ.

La composition tinctoriale selon l'invention peut être formulée à pH acide, neutre ou alcalin, le pH pouvant varier par exemple de 4 à 11 et, de préférence, de 5 à 10. Parmi les agents d'alcalinisation qui peuvent être utilisés, on peut mentionner les alcanolamines, les hydroxydes et les carbonates alcalins ou d'ammonium. Parmi les agents d'acidification qui peuvent être utilisés, on peut mentionner l'acide lactique, l'acide acétique, l'acide tartrique, l'acide phosphorique, l'acide chlorhydrique et l'acide citrique.

La composition tinctoriale selon l'invention peut contenir en outre divers adjuvants usuels tels que des agents anti-oxydants, des parfums, des agents séquestrants, des produits filmogènes et des agents de traitement, des agents dispersants, des agents de conditionnement du cheveu, des agents conservateurs, des agents opacifiants, ainsi que tout autre adjuvant utilisé habituellement en cosmétique.

La composition tinctoriale selon l'invention peut se présenter sous les diverses formes usuelles pour la teinture des cheveux, telles que des liquides épaissis ou gélifiés, des crèmes, des mousses en aérosols ou sous toutes autres formes appropriées pour réaliser une teinture de fibres kératiniques.

La présente invention a également pour objet un procédé de teinture des fibres kératiniques, et notamment des cheveux humains, consistant à laisser agir la composition tinctoriale ci-dessus définie sur les fibres kératiniques sèches ou humides. On peut utiliser la composition selon l'invention en tant que lotion non rincée, c'est-à-dire qu'on applique la composition selon l'invention sur les fibres kératiniques, puis on sèche sans rinçage intermédiaire. Dans les autres modes d'application, on applique la composition tinctoriale selon l'invention sur les fibres kératiniques pendant un temps de pose variant entre 3 et 60 minutes environ, de préférence entre 5 et 45 minutes environ, on rince, éventuellement on lave et on rince à nouveau, puis on sèche.

Les exemples qui suivent sont destinés à illustrer l'invention sans toutefois en limiter la portée.

### EXEMPLES DE PREPARATION

### Exemple 1: Préparation de la 2-nitro 5-méthylthio paraphénylènediamine

A une suspension de 25 g (0,35 mole) de thiométhylate de sodium dans 350 ml de diméthoxyéthane à température ambiante, on ajoute par portions 46,9 g (0,25 mole) de 2-nitro 5-chloro paraphénylènediamine.

La réaction est exothermique et on refroidit pour maintenir la température entre 25 °C et 30 °C.

A la fin de l'addition, on agite pendant une demi-heure et verse sur 2 kg d'eau glacée. Le précipité cristallisé est essoré, réempaté dans l'eau et séché.

On obtient des cristaux brun foncé (48,9 g) qui fondent à 168 °C (recristallisation de l'éthanol à 96°) et dont l'analyse élémentaire calculée pour C₇H₉N₃O₂S est :

| % | C | H | N | O | S |
|---|---|---|---|---|---|
| Calculé | 42,20 | 4,55 | 21,09 | 16,06 | 16,09 |
| Trouvé | 42,37 | 4,59 | 21,08 | 16,08 | 16,19 |

### Exemple 2: Préparation de la 2-nitro 5-éthylthio paraphénylènediamine

Ce composé est préparé selon le mode opératoire décrit pour l'exemple 1.

A partir de 18,7 g (0,1 mole) de 2-nitro-5-chloro-paraphénylènediamine 12,6 g (0,15 mole) de thioéthylate de sodium, on obtient des cristaux rouge brique (21,0 g) fondant à 142 °C (recristallisation de l'acétate d'éthyle) et dont l'analyse élémentaire calculée pour C₈H₁₁N₃O₂S est :

| % | C | H | N | O | S |
|---|---|---|---|---|---|
| Calculé | 45,06 | 5,20 | 19,70 | 15,00 | 15,04 |
| Trouvé | 45,17 | 5,20 | 19,74 | 15,14 | 15,14 |

### Exemple 3: Préparation de la 2-nitro 5-butylthio paraphénylènediamine

Ce composé est préparé selon le mode opératoire décrit pour l'exemple 1. A partir de 28,1 g (0,15 mole) de 2-nitro-5-chloro-paraphénylènediamine et 24,7 g (0,22 mole) de thiobutylate de sodium, on obtient 35,7 g de cristaux rouges fondant à 129 °C (recristallisation de l'acétate d'éthyle) et dont l'analyse élémentaire calculée pour C₁₀H₁₅N₃O₂S est :

| % | C | H | N | O | S |
|---|---|---|---|---|---|
| Calculé | 49,77 | 6,27 | 17,41 | 13,26 | 13,29 |
| Trouvé | 49,88 | 6,32 | 17,27 | 13,40 | 13,31 |

### Example 4 : Préparation de la 2-nitro 5-β-hydroxyéthylthio paraphénylènediamine

Une suspension de 18,7 g (0,1 mole) de 2-nitro-5-chloro-paraphénylènediamine, 20,7 g (0,15 mole) de carbonate de potassium et 11,7 g (0,15 mole) de 2-mercapto-éthanol dans 100 ml de diméthoxyéthane est chauffée au reflux pendant une heure.

Le milieu réactionnel est versé sur 600 g d'eau glacée. Le précipité cristallisé est essoré, réempaté dans l'eau et séché.

On obtient 22,5 g de cristaux grenat foncé fondant à 161 °C (recristallisation de l'éthanol à 96°) et dont l'analyse élémentaire calculée pour C₈H₁₁N₃O₃S est :

| % | C | H | N | O | S |
|---|---|---|---|---|---|
| Calculé | 41,91 | 4,84 | 18,33 | 20,94 | 13,99 |
| Trouvé | 42,04 | 4,91 | 18,19 | 21,22 | 13,76 |

### Exemple 5: Préparation de la 2-nitro 5-(β,γ-dihydroxypropyl)thio paraphénylènediamine

Ce composé est préparé selon le mode opératoire décrit pour l'exemple 4. A partir de 18,7 g (0,1 mole) de 2-nitro-5-chloro-paraphénylènediamine et 16,2 g (0,15 mole) de 3-mercapto-propane-1,2-diol, on obtient des cristaux brun rouge foncé (25,4 g) fondant à 164 °C (recristallisation de l'éthanol à 96°) et dont l'analyse élémentaire calculée pour C₉H₁₃N₃O₄S est :

| % | C | H | N | O | S |
|---|---|---|---|---|---|
| Calculé | 41,69 | 5,05 | 16,21 | 24,68 | 12,37 |
| Trouvé | 41,75 | 5,10 | 16,06 | 24,91 | 12,58 |

### Exemple 6: Préparation du 1,4-N-di-(β-hydroxyéthyl)amino-2-nitro 5-éthoxyéthylthio benzène

Ce composé est préparé selon le mode opératoire décrit pour l'exemple 4.

A partir de 8,3 g (0,03 mole) de 1,4-N-di-(β-hydroxyéthyl)amino 2-nitro 5-chloro benzène et 4,8 g (0,045 mole) de 2-éthoxy-éthanéthiol, on obtient des cristaux bruns (9,8 g) fondant à 110 °C (recristallisation de l'éthanol) et dont l'analyse élémentaire calculée pour C₁₄H₂₃N₃O₅S est :

| % | C | H | N | O | S |
|---|---|---|---|---|---|
| Calculé | 48,68 | 6,71 | 12,16 | 23,16 | 9,28 |
| Trouvé | 48,55 | 6,67 | 12,14 | 23,34 | 9,26 |

### Exemple 7: Préparation de la 2-nitro 4-N-(β-hydroxyéthyl)amino 5-β-hydroxyéthyl-thio aniline

Ce composé est préparé selon le mode opératoire décrit pour l'exemple 4. A partir de 23,1 g (0,1 mole) de 2-nitro 4-N-(β-hydroxyéthyl)amino 5-chloro aniline et de 11,7 g (0,15 mole) de 2-mercapto-éthanol, on obtient 21,7 g de cristaux brun orangé fondant à 166 °C et dont l'analyse élémentaire calculée pour C₁₀H₁₅N₃O₄S est :

| % | C | H | N | O | S |
|---|---|---|---|---|---|
| Calculé | 43,95 | 5,53 | 15,37 | 23,42 | 11,73 |
| Trouvé | 44,07 | 5,61 | 15,42 | 23,50 | 11,72 |

### Exemple 8: Préparation de la 2-nitro 5-(paraméthoxybenzyl)thio paraphénylènediamine

Ce composé est préparé selon le mode opératoire décrit pour l'exemple 4. A partir de 37,5 g (0,2 mole) de 2-nitro-5-chloro-paraphénylènediamine et 61,7 g (0,4 mole) de (4-méthoxyphényl)-méthanethiol, on obtient après recristallisation de l'éthanol à 96° des cristaux brun foncé (34,6 g) fondant à 140 °C et dont l'analyse élémentaire calculée pour C₁₄H₁₅N₃O₃S est :

| % | C | H | N | O | S |
|---|---|---|---|---|---|
| Calculé | 55,07 | 4,95 | 13,76 | 15,72 | 10,50 |
| Trouvé | 55,07 | 4,97 | 13,75 | 15,93 | 10,34 |

### Exemple 9: Préparation de la 2-nitro 4-N-(β-aminoéthyl)amino 5-méthylthio aniline

Ce composé est préparé selon le mode opératoire décrit pour l'exemple 1. A partir de 46,8 g (0,2 mole) de 2-nitro 4-N-(β-aminoéthyl)amino 5-chloro aniline et 25 g (0,35 mole) de thiométhylate de sodium, on obtient des cristaux rouge foncé (43,4 g) fondant à 153 °C (recristallisation de l'acétate d'isopropyle) et dont l'analyse élémentaire calculée pour C₉H₁₄N₄O₂S est :

| % | C | H | N | O | S |
|---|---|---|---|---|---|
| Calculé | 44,61 | 5,82 | 23,12 | 13,21 | 13,23 |
| Trouvé | 44,69 | 5,91 | 23,06 | 13,46 | 13,39 |

### Exemple 10: Préparation de la 2-nitro 4-N-(diéthylaminoéthyl)amino 5-méthylthio aniline

Ce composé est préparé selon le mode opératoire décrit pour l'exemple 1. A partir de 5,0 g (0,0174 mole) de 2-nitro 4-N-(diéthylaminoéthyl)amino 5-chloro aniline et 1,9 g (0,026 mole) de thiométhylate de sodium, on obtient des cristaux brun rouge (4,8 g) fondant à 105 °C (recristallisation de l'acétate d'isopropyle) et dont l'analyse élémentaire calculée pour C₁₃H₂₂N₄O₂S est :

| % | C | H | N | O | S |
|---|---|---|---|---|---|
| Calculé | 52,33 | 7,43 | 18,78 | 10,72 | 10,74 |
| Trouvé | 52,56 | 7,46 | 18,81 | 10,90 | 10,66 |

### Exemple 11: Préparation de la 2-nitro 4-N-(diéthylaminoéthyl)amino 5-éthylthio aniline

Ce composé est préparé selon le mode opératoire décrit pour l'exemple 1.

A partir de 5,0 g (0,0174 mole) de 2-nitro 4-N-(diéthylaminoéthyl)amino 5-chloro aniline et 2,2 g (0,026 mole) de thioéthylate de sodium, on obtient 5,1 g de cristaux brun rouge fondant à 61 °C et dont l'analyse élémentaire calculée pour C₁₄H₂₄N₄O₂S est :

| % | C | H | N | O | S |
|---|---|---|---|---|---|
| Calculé | 53,82 | 7,74 | 17,93 | 10,24 | 10,26 |
| Trouvé | 53,79 | 7,80 | 17,93 | 10,35 | 10,29 |

### Exemple 12: Préparation de la 2-nitro 4-N-(β,γ-dihydroxypropyl)amino 5-méthylthio aniline

Ce composé est préparé selon le mode opératoire décrit pour l'exemple 1.

A partir de 18,3 g (0,07 mole) de 2-nitro 4-N-(β,γ-dihydroxypropyl)amino 5-chloro aniline et de 9,8 g (0,14 mole) de thiométhylate de sodium, on obtient, après purification par passage sur une colonne moyenne pression de gel de silice (gradient d'acétate d'éthyle et d'heptane), des cristaux brun rouge (6,2 g) fondant à 133 °C et dont l'analyse élémentaire calculée pour C₁₀H₁₅N₃0₄S est :

| % | C | H | N | O | S |
|---|---|---|---|---|---|
| Calculé | 43,95 | 5,53 | 15,37 | 23,42 | 11,73 |
| Trouvé | 43,84 | 5,51 | 15,26 | 23,71 | 11,80 |

### Exemple 13: Préparation de la 2-nitro 4-N-(β,γ-dihydroxypropyl)amino 5-acétylaminoéthylthio aniline

On chauffe à 45 °C une suspension de 55,6 g (0,467 mole) de N-(2-mercapto-éthyl)acétamide et de 15,8 g (0,24 mole) de potasse en poudre à 85 % dans 300 ml de diméthoxyéthane jusqu'à dissolution de la potasse.

On ajoute par portions 49,0 g (0,187 mole) de 2-nitro 4-N-(β,γ-dihydroxypropyl)amino 5-chloro aniline en maintenant la température à 45 °C.

A la fin de l'addition, on agite pendant une heure et verse dans 1 litre d'eau glacée.

Le précipité cristallisé est essoré, réempaté dans l'eau et recristallisé de l'éthanol à 96° au reflux.

On obtient des cristaux bordeaux (32,9 g) fondant à 178 °C et dont l'analyse élémentaire calculée pour C₁₃H₂₀N₄O₅S est :

| % | C | H | N | O | S |
|---|---|---|---|---|---|
| Calculé | 45,34 | 5,85 | 16,27 | 23,23 | 9,31 |
| Trouvé | 45,38 | 5,91 | 16,24 | 22,99 | 9,27 |

### Exemple 14: Préparation de la 2-nitro 5-acétylaminoéthylthio paraphénylènediamine

Ce composé est préparé selon le mode opératoire décrit pour l'exemple 13. A partir de 46,9 g (0,25 mole) de 2-nitro-5-chloro-paraphénylènediamine et de 74,5 g (0,625 mole) de N-(2-mercapto-éthyl)-acétamide, on obtient 64,3 g de cristaux rouges (recristallisation de l'éthanol à 96°) fondant à 162 °C et dont l'analyse élémentaire calculée pour C₁₀H₁₄N₄0₃S est :

| % | C | H | N | O | S |
|---|---|---|---|---|---|
| Calculé | 44,43 | 5,22 | 20,73 | 17,76 | 11,86 |
| Trouvé | 44,56 | 5,24 | 20,64 | 17,72 | 11,81 |

### Exemple 15: Préparation du dichlorhydrate de 2-nitro 5-(β-aminoéthyl)thio paraphénylènediamine

On chauffe au reflux pendant 3 heures une solution de 20,3 g (0,075 mole) de 2-nitro 5-acétylaminoéthylthio paraphénylènediamine obtenue à l'exemple 14 dans 65 ml d'une solution aqueuse d'acide chlorhydrique à 36 %.

Le milieu réactionnel (suspension jaune) est refroidi dans un bain de glace et essoré. Après réempatage dans l'éthanol absolu et séchage, on obtient 8,3 g de cristaux jaunes fondant avec décomposition à 240-243 °C et dont l'analyse élémentaire calculée pour C₈H₁₂N₄O₂S, 2 HCl + 1/2 C₂H₅OH est :

| % | C | H | N | O | S | Cl |
|---|---|---|---|---|---|---|
| Calculé | 33,34 | 5,28 | 17,28 | 12,34 | 9,89 | 21,87 |
| Trouvé | 33,27 | 4,95 | 17,03 | 12,74 | 10,17 | 21,49 |

### Exemple 16 : Préparation du monohydrate de 2-nitro 5-[(diméthyl)aminoéthyl]thio paraphénylènediamine

On utilise le mode opératoire décrit pour l'exemple 4 avec deux modifications : le solvant est de la N-méthylpyrrolidone et la température de réaction est celle du bain-maire bouillant (95-100 °C).

A partir de 37,5 g (0,2 mole) de 2-nitro-5-chloro-paraphénylènediamine et de 42,5 g (0,3 mole) de chlorhydrate de 2-diméthylamino-éthanethiol, on obtient (après recristallisation de l'éthanol à 96°) 48,1 g de cristaux rouge orangé fondant à 122 °C et dont l'analyse élémentaire calculée pour C₁₀H₁₆N₄O₂S, H₂O est :

| % | C | H | N | O | S |
|---|---|---|---|---|---|
| Calculé | 43,78 | 6,61 | 20,42 | 17,50 | 11,69 |
| Trouvé | 44,04 | 6,59 | 20,45 | 17,00 | 11,97 |

### Exemple 17 : Préparation de la 2-nitro 4-N-(β-hydroxyéthyl)amino 5-[(diméthyl)aminoéthyl]thio aniline

Ce composé est préparé selon le mode opératoire décrit pour l'exemple 16. A partir de 23,1 g (0,1 mole) de 2-nitro 4-N-(β-hydroxyéthyl)amino 5-chloro aniline et de 21,2 g (0,15 mole) de chlorhydrate de 2-diméthylaminoéthanethiol, on obtient après recristallisation de l'éthanol à 96° des cristaux brun rouge (17,6 g) fondant à 133 °C et dont l'analyse élémentaire calculée pour C₁₂H₂₀N₄O₃S est

| % | C | H | N | O | S |
|---|---|---|---|---|---|
| Calculé | 47,98 | 6,71 | 18,65 | 15,98 | 10,67 |
| Trouvé | 48,01 | 6,77 | 18,74 | 15,94 | 10,57 |

### Exemple 18 : Préparation de la 2-nitro 5-(β-aminoéthyl)thio paraphénylènediamine

Ce composé est préparé selon le mode opératoire décrit pour l'exemple 16. A partir de 18,7 g (0,1 mole) de 2-nitro-5-chloro-paraphénylènediamine et de 17,0 g (0,15 mole) de chlorhydrate de 2-amino-éthanethiol, on obtient des cristaux brun-rouge (18,0 g) fondant à 149 °C (recristallisation de l'éthanol à 96°) et dont l'analyse élémentaire calculée pour C₈H₁₂N₄O₂S est :

| % | C | H | N | O | S |
|---|---|---|---|---|---|
| Calculé | 42,09 | 5,30 | 24,54 | 14,02 | 14,05 |
| Trouvé | 42,20 | 5,35 | 24,31 | 14,15 | 14,17 |

### Exemple 19: Préparation de la 2-nitro 4-amino 5-(β-hydroxypropyl)thio N-(méthyl)aniline

On utilise le mode opératoire décrit pour la préparation de l'exemple 4.

A partir de 11,5 g (0,057 mole) de 2-nitro 4-amino 5-chloro N-(méthyl)aniline et de 7,8 g (0,085 mole) de 1-mercapto-propan-2-ol, on obtient des cristaux brun foncé (14,3 g) fondant à 152 °C (recristallisation de l'éthanol à 96°) et dont l'analyse élémentaire calculée pour C₁₀H₁₅N₃O₃S est :

| % | C | H | N | O | S |
|---|---|---|---|---|---|
| Calculé | 46,68 | 5,88 | 16,33 | 18,65 | 12,46 |
| Trouvé | 46,61 | 5,91 | 16,38 | 18,81 | 12,26 |

### Exemple 20: Préparation de la 2-nitro 4-amino 5(para-hydroxyphényl)thio N-(méthyl) aniline

Ce composé est préparé selon le mode opératoire décrit pour l'exemple 4.

A partir de 11,5 g (0,057 mole) de 2-nitro-4-amino Schloro N-(méthyl) aniline et de 11,9 g (0,085 mole) de 4-mercapto-phénol, on obtient des cristaux brun-orangé (11,3 g) recristallisés de l'éthanol à 96°, fondant à 206 °C et dont l'analyse élémentaire calculée pour C₁₃H₁₃N₃O₃S est :

| % | C | H | N | O | S |
|---|---|---|---|---|---|
| Calculé | 53,60 | 4,50 | 14,42 | 16,48 | 11,01 |
| Trouvé | 53,41 | 4,58 | 14,21 | 16,95 | 10,90 |

### Exemple 21: Préparation de la 2-nitro 5-éthoxycarbonyléthylthio paraphénylènediamine

Ce composé est préparé selon le mode opératoire décrit pour l'exemple 16.

A partir de 22,5 g (0,12 mole) de 2-nitro-5-chloro-paraphénylènediamine et de 25,0 g (0,186 mole) d'éthoxycarbonyléthylthiol, on obtient des cristaux mordorés (21,6 g) dont le point de fusion est 120°C et dont l'analyse élémentaire calculée pour C₁₁H₁₅N₃O₄S est

| % | C | H | N | O | S |
|---|---|---|---|---|---|
| Calculé | 46,31 | 5,30 | 14,73 | 22,43 | 11,24 |
| Trouvé | 46,31 | 5,41 | 14,66 | 22,23 | 11,41 |

### Exemple 22 : Préparation de la 2-nitro 5-méthylsulfinyl paraphénylènediamine

A une suspension refroidit à 0°C de 19,9 g (0,1 mole) de 2-nitro-5-méthylthio paraphénylénediamine (obtenu à l'exemple 1) dans 300 ml d'acétone, on ajoute au goutte à goutte rapide et en maintenant la température à + 10 °C une solution de 35,3 g (0,115 mole) de monopersulfate de potassium (triple sel) dans 300 ml d'eau. On agite une heure à + 10 °C, ajoute 300 g de glace et neutralise avec une solution diluée de soude.

On extrait à l'acétate d'éthyle, sèche sur sulfate de sodium et évapore à sec sous pression réduite.

Après purification par passage sur colonne de gel de silice (gradient d'heptane et d'acétate d'éthyle), on obtient des cristaux rouge foncé (7,9 g) fondant à 204 °C et dont l'analyse élémentaire calculée pour C₇H₉N₃O₃S est :

| % | C | H | N | O | S |
|---|---|---|---|---|---|
| Calculé | 39,06 | 4,21 | 19,52 | 22,30 | 14,90 |
| Trouvé | 39,20 | 4,28 | 19,58 | 22,31 | 14,83 |

### Exemple 23 : Préparation de la 2-nitro 5-mésyl paraphénylènediamine

### 1ère étape

Synthèse du 1,4-di-N-acétylamino 2-nitro 5-méthylthiobenzène.

On chauffe une heure au bain-marie bouillant un mélange de 59,7 g (0,3 mole) de 2-nitro 5-méthylthio paraphénylènediamine (obtenu à l'exemple 1) et de 66 ml d'anhydride acétique dans 350 ml de dioxane.

On refroidit la suspension jaune dans un bain de glace.

Le précipité cristallisé est essoré, lavé au dioxane puis à l'éther de pétrole et séché.

On obtient des cristaux jaunes (71,7 g) fondant à 247 °C et dont l'analyse élémentaire calculée pour C₁₁H₁₃N₃O₄S est :

| % | C | H | N | O | S |
|---|---|---|---|---|---|
| Calculé | 46,64 | 4,63 | 14,83 | 22,59 | 11,32 |
| Trouvé | 46,68 | 4,64 | 14,89 | 22,60 | 11,14 |

### 2ème étape

A une suspension de 38,0 g (0,134 mole) du composé obtenu ci-dessus à l'étape 1 dans 700 ml d'acétone, on ajoute rapidement à température ambiante une solution de 103 g (0,335 mole) de monopersulfate de potassium (triple sel) dans 700 ml d'eau en laissant la température monter à 30 °C.

On agite 2 heures à température ambiante et dilue avec 700 g d'eau glacée.

Le précipité cristallisé est essoré, réempaté dans l'eau et séché.

On obtient des cristaux jaunes (36,4 g) qui fondent à 256 °C (recristallisation de l'acide acétique) et dont l'analyse élémentaire calculée pour C₁₁H₁₃N₃O₆S est :

| % | C | H | N | O | S |
|---|---|---|---|---|---|
| Calculé | 41,90 | 4,16 | 13,33 | 30,45 | 10,17 |
| Trouvé | 42,06 | 4,25 | 13,17 | 30,54 | 10,22 |

### 3ème étape

Le composé obtenu ci-dessus (étape 2 - 36,4 g) est désacétylé par chauffage au bain-marie bouillant pendant 30 mn dans un mélange de 200 ml d'acide chlorhydrique aqueux à 36 % et de 40 ml d'acide acétique.

On refroidit dans un bain de glace.

Le chlorhydrate cristallisé est essoré et repris dans 200 ml d'eau ammoniacale.

Le précipité cristallisé est essoré, réempaté dans l'eau et séché.

On obtient des cristaux bruns violets (12,8 g) du produit attendu fondant à 221 °C et dont l'analyse élémentaire calculée pour C₇H₉N₃O₄S est :

| % | C | H | N | O | S |
|---|---|---|---|---|---|
| Calculé | 36,36 | 3,92 | 18,17 | 27,68 | 13,87 |
| Trouvé | 36,87 | 4,01 | 18,05 | 27,52 | 13,89 |

### Exemple 24: Préparation du méthylsulfate de 2-nitro 5-triméthylammonioéthylthio paraphénylènediamine

A une suspension à température ambiante de 13,7 g (0,05 mole) de 2-nitro 5-(diméthylamino éthylthio) paraphénylènediamine monohydrate préparé à l'exemple 16 dans 250 ml d'acétate d'éthyle, on ajoute 4,8 ml (0,05 mole) de diméthyle sulfate.

On agite pendant 4 heures à température ambiante, essore le précipité cristallisé et lave à l'acétate d'éthyle.

Après séchage, on obtient des cristaux brun rouge foncé (18,8 g) fondant à 153-154 °C et dont l'analyse élémentaire calculée pour C₁₁H₁₉N₄O₂S, CH₃O₄S est :

| % | C | H | N | O | S |
|---|---|---|---|---|---|
| Calculé | 37,69 | 5,80 | 14,65 | 25,10 | 16,77 |
| Trouvé | 37,72 | 5,71 | 14,52 | 24,89 | 16,98 |

### Exemple 25: Préparation du monochlorhydrate 1-N-(β-hydroxyéthyl)amino 2-nitro 4,N,N-bis-(β-hydroxyéthyl)amino 5-isopropylthio benzène

### 1ère étape

Synthèse du 2-nitro 4-N,N-(di-β-hydroxyéthyl)amino 1-N-(β-hydroxyéthyl)amino 5-chloro benzène.

On chauffe 12 heures au bain-marie bouillant une suspension de 18,7 g (0,1 mole) de 2-nitro-5-chloro-paraphénylènediamine, de 30 g de carbonate de calcium et de 62,5 g (0,5 mole) de 2-bromo-éthanol dans 50 ml d'eau.

On ajoute trois volumes d'eau glacée au milieu réactionnel, extrait à l'acétate d'éthyle, sèche sur sulfate de sodium, filtre et évapore à sec sous pression réduite.

L'huile orangée obtenue est purifiée par passage sur colonne de gel de silice (gradient d'heptane et d'acétate d'éthyle).

On obtient des cristaux jaune orangé qui fondent à 94 °C et dont l'analyse élémentaire calculée pour C₁₂H₁₈ClN₃0₅ est :

| % | C | H | Cl | N | O |
|---|---|---|---|---|---|
| Calculé | 45,08 | 5,67 | 11,09 | 13,14 | 25,02 |
| Trouvé | 45,11 | 5,71 | 10,83 | 13,05 | 25,02 |

### 2ème étape

Le composé obtenu ci-dessus à la 1ère étape (9,6 g - 0,03 mole) et 5,9 g (0,06 mole) de thioisopropylate de sodium sont mis en réaction selon le mode opératoire décrit pour l'exemple 1.

On obtient, après purification et passage au chlorhydrate dans l'éthanol absolu, des cristaux jaunes fondant à 162-164 °C et dont l'analyse élémentaire calculée pour C₁₅H₂₅N₃O₅S, HCl est :

| % | C | H | N | O | S | Cl |
|---|---|---|---|---|---|---|
| Calculé | 45,51 | 6,62 | 10,61 | 20,21 | 8,10 | 8,95 |
| Trouvé | 45,32 | 6,52 | 10,61 | 20,01 | 8,34 | 8,66 |

### Exemple 26: Préparation du monohydrate de 2-nitro 5-sulfoéthylthio paraphénylènediamine

On utilise le mode opératoire décrit pour l'exemple 16, en remplaçant le carbonate de potassium par du carbonate de sodium.

A partir de 18,7 g (0,1 mole) de 2-nitro-5-chloro-paraphénylènediamine et de 24,6 g (0,15 mole) de sel de sodium de l'acide 2-mercapto-éthanesulfonique, on obtient des cristaux jaune orangé (27,6 g) fondant à plus de 260 °C et dont l'analyse élémentaire calculée pour C₈H₁₁N₃O₅S₂, H₂O est :

| % | C | H | N | O | S |
|---|---|---|---|---|---|
| Calculé | 30,86 | 4,21 | 13,50 | 30,83 | 20,60 |
| Trouvé | 30,97 | 4,18 | 13,42 | 30,14 | 20,60 |

### EXEMPLES DE COMPOSITIONS TINCTORIALES

### EXEMPLE 27

On prépare la composition tinctoriale suivante :
- méthylsulfate de 2-nitro 5-triméthylammonioéthylthio paraphénylènediamine 0,25 g
- N-(β-hydroxyéthyl)amino-3-nitro 4-amino benzène 0,1 g
- 1,4-di-N-(β,γ-dihydroxypropyl)amino anthraquinone 0.05 g
- N-(β-hydroxyéthyl)amino-2-nitro paraphénylènediamine 0,02 g
- monométhyléther de propylène glycol 10 g
- nonylphénol oxyéthylèné à 9 moles d'oxyde d'éthylène vendu sous la dénomination RHODIASURF NP 9 OR par la société RHONE POULENC 8 g
- diéthanolamine de coprah vendu sous la dénomination COMPERLAN KD par la société HENKEL 2 g
- acide citrique q.s pH 9
- eau déminéralisée q.s.p 100 g
On applique la composition ci-dessus sur des mèches de cheveux gris naturels à 90 % de blancs. On laisse poser 30 minutes à température ambiante. Après rinçage et séchage, les cheveux sont teints dans une couleur blond foncé irisé.

### EXEMPLE 28

On prépare la composition tinctoriale suivante :
- 2-nitro 4-N-(β-hydroxyéthyl)amino 5-[(diméthyl)aminoéthyl]thioparaphénylènediamine 0,3 g
- 1-β-hydroxyéthyloxy-3-méthylamino-4-nitrobenzène 0,05 g
- 1-hydroxy-3-nitro-4-aminobenzène 0,03 g
- 1-(β-aminoéthylamino)-2-nitro-5-méthoxybenzène 0,1 g
- monométhyléther de propylène glycol 10 g
- nonylphénol oxyéthylèné à 9 moles d'oxyde d'éthylène vendu sous la dénomination RHODIASURF NP 9 OR par la société RHONE POULENC 8 g
- diéthanolamine de coprah vendu sous la dénomination COMPERLAN KD par la société HENKEL 2 g
- 2-amino-2-méthyl-1-propanol q.s pH 9
- eau déminéralisée q.s.p 100 g
On applique la composition ci-dessus sur des mèches de cheveux gris naturels à 90 % de blancs. On laisse poser 30 minutes à température ambiante. Après rinçage et séchage, les cheveux sont teints dans une couleur blond clair cuivré doré.

### EXEMPLE 29

On prépare la composition tinctoriale suivante :
- dichlorhydrate de 2-nitro 5-(β-aminoéthyl)thio paraphénylènediamine 0,22 g
- 1-hydroxy-3-nitro-4-(γ-hydroxypropylamino)benzène 0,02 g
- 4-(β-hydroxyéthyl)amino-3-nitro-méthylbenzène 0,03 g
- 1-N,N-(bis-β-hydroxyéthyl)amino-3-méthyl-4-N-(4'-aminophénylazo)aminobenzène 0,05 g
- monométhyléther de propylène glycol 10 g
- nonylphénol oxyéthylèné à 9 moles d'oxyde d'éthylène vendu sous la dénomination RHODIASURF NP 9 OR par la société RHONE POULENC 8 g
- diéthanolamine de coprah vendu sous la dénomination COMPERLAN KD par la société HENKEL 2 g
- 2-amino-2-méthyl-1-propanol q.s pH 9
- eau déminéralisée q.s.p 100 g

On applique la composition ci-dessus sur des mèches de cheveux gris naturels à 90 % de blancs. On laisse poser 30 minutes à température ambiante. Après rinçage et séchage, les cheveux sont teints dans une couleur blond cuivré irisé.

### EXEMPLE 30

On prépare la composition tinctoriale suivante :
- 2-nitro 4-N-(β,γ-dihydroxypropyl)amino 5-méthylthio aniline 0,15 g
- 1-N-(β-hydroxyéthyl)amino-4-(N-éthyl, N-β-hydroxyéthyl)amino-2-nitrobenzène 0,1 g
- 1-(β-aminoéthyl)amino-2-nitro 4-(β-hydroxyéthyl)oxybenzène 0,07 g
- monométhyléther de propylène glycol 10 g
- nonylphénol oxyéthylèné à 9 moles d'oxyde d'éthylène vendu sous la dénomination RHODIASURF NP 9 OR par la société RHONE POULENC 8 g
- diéthanolamine de coprah vendu sous la dénomination COMPERLAN KD par la société HENKEL 2 g
- acide citrique q.s pH 9
- eau déminéralisée q.s.p 100 g

On applique la composition ci-dessus sur des mèches de cheveux gris naturels à 90 % de blancs. On laisse poser 30 minutes à température ambiante. Après rinçage et séchage, les cheveux sont teints dans une couleur blond clair cuivré irisé.

## Revendications

1. 2-nitro paraphénylénediamine soufrée en position 5 ayant pour formule : 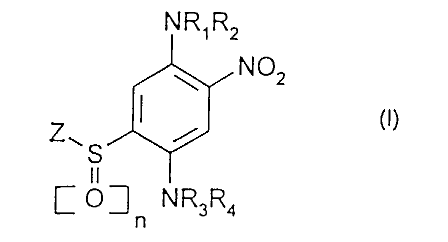 dans laquelle :
- n = 0, 1 ou 2 ;
- R₁, R₂, R₃, R₄ identiques ou différents représentent un atome d'hydrogène, un radical alkyle linéaire ou ramifié en C₁-C₄, monohydroxyalkyle en C₁-C₄, polyhydroxyalkyle en C₂-C₄, aminoalkyle en C₁-C₄, mono ou dialkyl(C₁-C₄)aminoalkyle en C₁-C₄ ;
- Z représente un radical alkyle linéaire ou ramifié en C₁-C₄, monohydroxyalkyle en C₁-C₄, polyhydroxyalkyle en C₂-C₄, aminoalkyle en C₁-C₄, alcoxy(C₁-C₄)alkyle en C₁-C₄, acétylaminoalkyle en C₁-C₄, alkyl alkyl(C₁-C₄)aminoalkyle en C₁-C₄, dialkyl(C₁-C₄)aminoalkyle en C₁-C₄, trialkyl trialkyl(C₁-C₄)ammonioalkyle en C₁-C₄ d'un halogénure d'alkyle en C₁-C₄ ou d'un alkylsulfate en C₁-C₄, benzyle éventuellement substitué, phényle éventuellement substitué, sulfoalkyle en C₁-C₄, alkoxy alkoxy(C₁-C₄)carbonylalkyle en C₁C₄, et les sels cosmétiquement acceptables de ces composés.

2. Composé selon la revendication 1, caractérisé par le fait que les radicaux alkyle, hydroxyalkyle ou alcoxyalkyle désignent méthyle, éthyle, propyle, butyle, hydroxyéthyle, hydroxypropyle, dihydroxypropyle ou éthoxyéthyle.

3. Composé selon la revendication 1 ou 2, caractérisé par le fait que le radical R₁ représente un atome d'hydrogène.

4. Composé selon l'une quelconque de revendications 1 à 3, caractérisé par le fait qu'il est choisi parmi les composés suivants :
- la 2-nitro 5-triméthylammonioéthylthio paraphénylènediamine,
- la 2-nitro 4-N-(β-hydroxyéthyl)amino 5-[(diméthyl)aminoéthyl]thio-aniline
- la 2-nitro 5-(β-aminoéthyl)thio paraphénylénediamine,
- la 2-nitro 4-N-(β,γ-dihydroxypropyl)amino 5-méthylthio aniline,
- la 2-nitro 5-méthylthio paraphénylènediamine,
- la 2-nitro 5-éthylthio paraphénylénediamine,
- la 2-nitro 5-butylthio paraphénylénediamine,
- la 2-nitro 5-β-hydroxyéthylthio paraphénylènediamine,
- la 2-nitro 5-(β,γ-dihydroxypropyl)thio paraphénylènediamine,
- le 1,4-N-di-(β-hydroxyéthyl)amino 2-nitro 5-éthoxyéthylthio benzène,
- la 2-nitro 4-N- 5-β-hydroxyéthylthio )amino 5-β-hydroxyéthylthio aniline,
- la 2-nitro 5-(paraméthoxybenzyl)thio paraphénylènediamine,
- la 2-nitro 4-N-(β-aminoéthyl)amino 5-méthylthio aniline,
- la 2-nitro 4-N-(diéthylaminoéthyl)amino 5-méthylthio aniline,
- la 2-nitro 4-N-(diéthylaminoéthyl)amino 5-éthylthio aniline,
- la 2-nitro 4-N-(β,γ-dihydroxypropyl)amino 5-acétylaminoéthylthio aniline,
- la 2-nitro 5-acétylaminoéthylthio paraphénylénediamine,
- la 2-nitro 5-[(diméthyl)aminoéthyl]thio paraphénylénediamine,
- la 2-nitro 5-(β-aminoéthyl)thio paraphénylénediamine,
- la 2-nitro 4-amino 5-(β-hydroxypropyl)thio N-(méthyl)aniline,
- la 2-nitro 4-amino 5-(para-hydroxyphényl)thio N-(méthyl) aniline,
- la 2-nitro 5-éthoxycarbonyléthylthio paraphénylénediamine,
- la 2-nitro 5-méthylsulfinyl paraphénylènediamine,
- la 2-nitro 5-mésyl paraphénylénediamine,
- le 1-N-(β-hydroxyéthyl)amino 2-nitro 4,N,N-bis-(β-hydroxyéthyl)amino 5-isopropylthiobenzène,
- la 2-nitro 5-sulfoéthylthio paraphénylénediamine,
et les sels cosmétiquement acceptables de ces composés.

5. Procédé de préparation d'un composé de formule (I) selon l'une quelconque des revendications 1 à 4, caractérisé par le fait qu'il consiste, dans une première étape, à faire réagir en milieu solvant, à une température comprise entre la température ambiante et la température de reflux du milieu réactionnel, en présence d'un capteur de l'acide halohydrique, un composé de formule (Il) 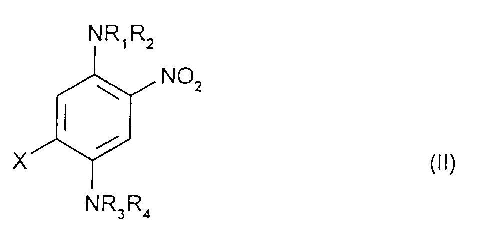 dans laquelle R₁, R₂, R₃ et R₄ ont les significations indiquées dans la revendication 1 et X représente un atome d'halogène choisi parmi le chlore, le brome et l'iode, avec un thiol de formule (III)
ASZ (III)
dans laquelle Z a les significations indiquées dans la revendication 1 et A est un atome d'hydrogène, de sodium ou de potassium, pour obtenir un composé de formule (I) dans lequel n = 0 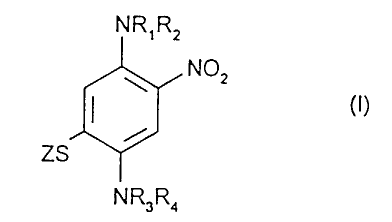 Dans une deuxième étape, le composé de formule (I) dans lequel n = 0 est éventuellement oxydé en milieu solvant, en présence d'un agent oxydant du soufre tel que le monopersulfate de potassium, à une température comprise entre zéro et 40°C, pour obtenir un composé de formule (I) dans lequel n = 1 ou 2.

6. procédé de préparation selon la revendication 5 caractérisé par le fait que le solvant est choisi parmi le diméthoxy 1,2-éthane, le diméthylformamide, la N-méthylpyrrolidone et le dioxane.

7. Procédé de préparation selon la revendication 5 ou 6 caractérisé par le fait que le capteur de l'acide halohydrique est choisi parmi la soude, la potasse, la triéthylamine, les carbonates de sodium, de potassium et de calcium, et les sels du thiol de formule (III) utilisés.

8. Composition tinctoriale pour la coloration directe des fibres kératiniques et en particulier des cheveux humains, caractérisée par le fait qu'elle contient dans un véhicule aqueux, alcoolique ou hydroalcoolique, au moins un composé de formule (I) : 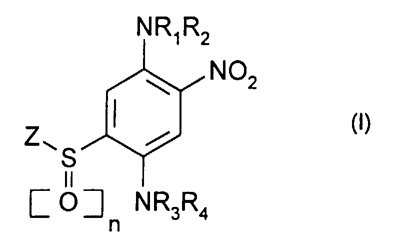 dans laquelle :
- n = 0, 1 ou 2 ;
- R₁, R₂, R₃, R₄ identiques ou différents représentent un atome d'hydrogène, un radical alkyke linéaire ou ralifié en C₁-C₄, monohydroxyalkyle en C₁-C₄, polyhydroxyalkyle en C₂-C₄, aminoalkyle en C₁-C₄, mono ou dialkyl(C₁-C₄)aminoalkyle en C₁-C₄;
- Z représente un radical alkyle linéaire ou ramifié en C₁-C_{4'}, monohydroxyalkyle en C₁-C₄, polyhydroxyalkyle en C₂-C₄, aminoalkyle en C₁-C₄, alcoxy(C₁-C₄)alkyle en C₁-C₄, acétylaminoalkyle en C₁-C₄, alkyl(C₁-C₄)aminoalkyle en C₁-C₄, dialkyl(C₁-C₄)aminoalkyle en C₁-C₄, trialkyl(C₁-C₄)ammonioalkyle en C₁-C₄ d'un halogènure d'alkyle en C₁-C₄ ou d'un alkylsulfate en C₁-C₄, benzyle éventuellement substitué, phényle éventuellement substitué, sulfoalkyle en C₁-C₄, alkoxy(C₁-C₄)carbonylalkyle en C₁-C₄; ou l'un de ses sels cosmétiquement acceptables.

9. Composition tinctoriale selon la revendication 8 caractérisée par le fait que les radicaux alkyle, hydroxyalkyle ou alcoxy du composé de formule (I) désignent méthyle, éthyle, propyle, butyle, hydroxyéthyle, hydroxypropyle, dihydroxypropyle ou éthoxyéthyle.

10. Composition tinctoriale selon la revendication 8 ou 9 caractérisée par le fait que le radical R₁ du composé de formule (I) est un atome d'hydrogène.

11. Composition tinctoriale selon l'une quelconque des revendications 8 à 10 caractérisée par le fait qu'elle contient un composé de formule (I) choisi parmi :
- la 2-nitro 5-triméthylammonioéthylthio paraphénylènediamine,
- la 2-nitro 4-N-(β-hydroxyéthyl)amino 5-[(diméthyl)aminoéthyl]thio-aniline
- la 2-nitro 5-(β-aminoéthyl)thio paraphénylènediamine,
- la 2-nitro 4-N-(β,γ-dihydroxypropyl)amino 5-méthylthio aniline,
- la 2-nitro 5-méthylthio paraphénylénediamine,
- la 2-nitro 5-éthylthio paraphénylènediamine,
- la 2-nitro 5-butylthio paraphénylènediamine,
- la 2-nitro 5-β-hydroxyéthylthio paraphénylènediamine,
- la 2-nitro 5-(β,γ-dihydroxypropyl)thio paraphénylènediamine,
- le 1,4-N-di-(β-hydroxyéthyl))amino 2-nitro 5-éthoxyéthylthio benzène,
- la 2-nitro 4-N-(β-hydroxyéthyl)amino 5-β-hydroxyéthylthio aniline,
- la 2-nitro 5-(paraméthoxybenzyl)thio paraphénylénediamine,
- la 2-nitro 4-N-(β-aminoéthyl)amino 5-méthylthio aniline,
- la 2-nitro 4-N-(diéthylaminoéthyl)amino 5-méthylthio aniline,
- la 2-nitro 4-N-(diéthylaminoéthyl)amino 5-éthylthio aniline,
- la 2-nitro 4-N-(β,γ-dihydroxypropyl)amino 5-acétylaminoéthylthio aniline,
- la 2-nitro 5-acétylaminoéthylthio paraphénylènediamine,
- la 2-nitro 5-[(diméthyl)aminoéthyl]thio paraphénylénediamine,
- la 2-nitro 5-(β-aminoéthyl)thio paraphénylènediamine,
- la 2-nitro 4-amino 5-(β-hydroxypropyl)thio N-(méthyl)aniline,
- la 2-nitro 4-amino 5-(para-hydroxyphényl)thio N-(méthyl) aniline,
- la 2-nitro 5-éthoxycarbonyléthylthio paraphénylènediamine,
- la 2-nitro 5-méthylsulfinyl paraphénylénediamine,
- la 2-nitro 5-mésyl paraphénylénediamine,
- le 1-N-(β-hydroxyéthyl)amino 2-nitro 4,N,N-bis-(β-hydroxyéthyl)amino 5-isopropylthiobenzène,
- la 2-nitro 5-sulfoéthylthio paraphénylènediamine,
et les sels cosmétiquement acceptables de ces composés.

12. Composition tinctoriale selon l'une quelconque des revendications 8 à 11, caractérisée par le fait qu'elle contient au moins un colorant nitré benzénique jaune ou jaune vert de nuance selon MUNSELL comprise entre 2,4 Y et 0,2 YR sur cheveux gris à 90 % de blancs.

13. Composition tinctoriale selon la revendication 12 caractérisée par le fait que le colorant nitré benzénique jaune ou jaune vert est choisi parmi les composés suivants :
- le 1-β-hydroxyéthyloxy-3-méthylamino-4-nitrobenzène,
- le 1-(méthylamino)-2-nitro-5-(β,γ-dihydroxypropyl)oxybenzène,
- le 1-(β-hydroxyéthylamino)-2-méthoxy-4-nitrobenzène,
- le 1-(β-aminoéthylamino)-2-nitro-5-méthoxy-benzène,
- le 1,3-di-(β-hydroxyéthylamino)-4-nitro-6-chlorobenzène,
- le 1-amino-2-nitro-6-méthyl-benzène,
- le 1-(β-hydroxyéthylamino)-2-hydroxy-4-nitrobenzène,
- la N-(β-hydroxyéthyl)-2-nitro-4-trifluorométhylaniline,
- l'acide 4-β-hydroxyéthylamino-3-nitro-benzènesulfonique,
- l'acide 4-éthylamino-3-nitro-benzoïque,
- le 4-(β-hydroxyéthyl)amino-3-nitro-chlorobenzène,
- le 4-(β-hydroxyéthyl)amino-3-nitro-méthylbenzène,
- le 4-(β, γ-dihydroxypropyl)amino-3-nitro-trifluorométhylbenzène,
- le 1-β-uréidoéthylamino-4-nitrobenzène,
- l'O,N-bis(β-hydroxyéthyl)-2-amino-5-nitrophénol,
- le 1,3-diamino-4-nitrobenzène,
- le 1-hydroxy-2-amino-5-nitrobenzène,
- le 1-amino-2-[tris(hydroxyméthyl)méthyl]amino-5-nitro-benzène,
- le 1-(β-hydroxyéthyl)amino-2-nitrobenzène,
- le 4-(β-hydroxyéthylamino)-3-nitrobenzamide.

14. Composition tinctoriale selon l'une quelconque des revendications 8 à 13 caractérisée par le fait qu'elle contient au moins un colorant nitré benzénique bleu choisi parmi les composés suivants :
- le 1-(β-hydroxyéthyl)amino-4-N,N-bis(β-hydroxyéthyl)amino 2-nitrobenzène,
- le 1-(γ-hydroxypropyl)amino-4-N,N-bis(β-hydroxyéthyl)amino 2-nitrobenzène,
- le 1-(β-hydroxyéthyl)amino-4-(N-méthyl, N-β-hydroxyéthyl)amino 2-nitrobenzène,
- le 1-(β-hydroxyéthyl)amino-4-(N-éthyl, N-β-hydroxyéthyl)amino 2-nitrobenzène,
- le 1-(β, γ-dihydroxypropyl)amino-4-(N-éthyl, N-β-hydroxyéthyl)amino 2-nitrobenzène,
- les 2-nitroparaphénylènediamines de formule : 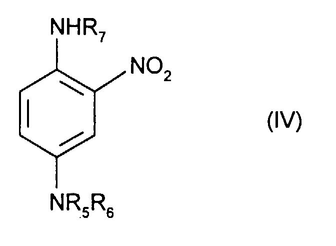
dans laquelle :
- R₅ représente un radical alkyle en C₁-C₄, β-hydroxyéthyle, β-hydroxypropyle ou γ-hydroxypropyle;
- R₆ et R₇, indépendamment l'un de l'autre, représentent un radical β-hydroxyéthyle, β-hydroxypropyle,γ-hydroxypropyle, ou β,γ-dihydroxypropyle, l'un au moins des radicaux R₅, R₆ ou R₇ représentant un radical y-hydroxypropyle et R₅ et R₆ ne pouvant désigner simultanément un radical β-hydroxyéthyle lorsque R₇ est un radical γ-hydroxypropyle, tels que décrits dans la demande de brevet FR 92-07515.

15. Composition tinctoriale selon l'une quelconque des revendications 8 à 14 caractérisée par le fait qu'elle contient au moins un colorant nitré benzénique rouge choisi parmi les composés suivants :
- le 1-hydroxy-3-nitro-4-(γ-hydroxypropylamino)benzène,
- le N-(β-hydroxyéthyl)amino-3-nitro-4-aminobenzène,
- le 1-amino-3-méthyl-4-(β-hydroxyéthyl)amino-6-nitrobenzène,
- le 1-hydroxy-3-nitro-4-N-β-hydroxyéthyl aminobenzène,
- le 1,4-diamino-2-nitrobenzène,
- le 1-amino-2-nitro-4-méthylaminobenzène,
- la N-(β-hydroxyéthyl)-2-nitro-paraphénylènediamine,
- le 1-amino-2-nitro-4-(β-hydroxyéthylamino)-5-chlorobenzène,
- la 2-nitro-4-amino-diphénylamine,
- le 1-amino-3-nitro-6-hydroxybenzène.

16. Composition tinctoriale selon l'une quelconque des revendications 8 à 15 caractérisée par le fait qu'elle contient au moins un colorant nitré benzénique orangé choisi parmi les composés suivants :
- le 1-(β-aminoéthyl)amino-2-nitro-4-(β-hydroxyéthyl)oxybenzène,
- le 1-(β,γ-dihydroxypropyl)oxy-3-nitro-4-(β-hydroxyéthyl)aminobenzène,
- le 1-hydroxy-3-nitro-4-aminobenzène,
- le 1-hydroxy-2-amino-4,6-dinitrobenzène,
- le 1-méthoxy-3-nitro-4-(β-hydroxyéthylamino)benzène,
- la 2-nitro-4'-hydroxydiphénylamine,
- le 1-amino-2-nitro-4-hydroxy-5-méthylbenzène.

17. Composition tinctoriale selon l'une quelconque des revendications 8 à 16, caractérisée par le fait qu'elle contient d'autres colorants directs choisis parmi les colorants azoïques, anthraquinoniques, les dérivés du triarylméthane et les colorants basiques.

18. Composition tinctoriale selon l'une quelconque des revendications 8 à 17 caractérisée par le fait qu'elle contient le composé de formule (I) en des proportions comprises entre 0,01 et 10 % en poids, exprimées en base libre, par rapport au poids total de la composition.

19. Composition tinctoriale selon l'une quelconque des revendications 12 à 18 caractérisée par le fait qu'elle contient en outre de 0,05 à 3 % en poids de colorants nitrés benzéniques jaune et/ou jaune vert et/ou bleu par rapport au poids total de la composition.

20. Composition tinctoriale selon l'une quelconque des revendications 15 à 19 caractérisée par le fait qu'elle contient en outre 0,05 à 10 % en poids d'autres colorants directs.

21. Composition tinctoriale selon l'une quelconque des revendications 8 à 20 caractérisée par le fait qu'elle contient au moins un solvant organique choisi parmi les alcools, les glycols et les éthers de glycols en des proportions comprises entre 0,5 % et 20 % par rapport au poids total de la composition.

22. Composition tinctoriale selon l'une quelconque des revendications 8 à 21 caractérisée par le fait qu'elle contient au moins un adjuvant choisi parmi les amides gras, les agents tensioactifs anioniques, cationiques, non ioniques, amphotères, zwittérioniques et leurs mélanges, les épaississants, les agents anti-oxydants, les parfums, les agents séquestrants, les agents filmogènes, les agents de traitement du cheveu, les agents dispersants, les agents de conditionnement du cheveu, les agents conservateurs et les agents opacifiants.

23. Composition tinctoriale selon l'une quelconque des revendications 8 à 22 caractérisée par le fait qu'elle a un pH compris entre 4 et 11.

24. Procédé de teinture des fibres kératiniques, et notamment des cheveux humains par coloration directe, caractérisé par le fait qu'on applique la composition tinctoriale selon l'une quelconque des revendications 8 à 23 sur les fibres kératiniques sèches ou humides, et on sèche ces fibres kératiniques sans rinçage intermédiaire.

25. Procédé de teinture des fibres kératiniques, et notamment des cheveux humains par coloration directe, caractérisé par le fait qu'on applique la composition tinctoriale selon l'une quelconque des revendications 8 à 23 sur les fibres kératiniques sèches ou humides et qu'après avoir laissé agir la composition pendant 3 à 60 minutes, de préférence pendant 5 à 45 minutes, on rince les fibres kératiniques, on les lave éventuellement et on les rince à nouveau, puis on les sèche.

## Patentansprüche

1. 2-Nitro-p-phenylendiamine, die in 5-Stellung ein Schwefelatom aufweisen und die folgender Formel entsprechen: 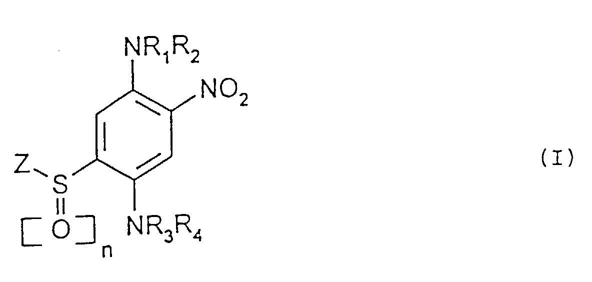 und worin bedeuten:
- n = 0, 1 oder 2;
- R₁, R₂, R₃ und R₄, die gleich oder voneinander verschieden sind, ein Wasserstoffatom, eine geradkettige oder verzweigte C₁₋₄-ASky-gruppe, eine C₁₋₄-Monohydroxyalkylgruppe, eine C₂₋₄-Polyhydroxyalkylgruppe, eine C₁₋₄-Aminoalkylgruppe und eine Mono- oder Dialkylaminoalkylgruppe, wobei die Alkylgruppen 1 bis 4 Kohlenstoffatome aufweisen;
- Z eine geradkettige oder verzweigte C₁₋₄-Alkylgruppe, C₁₋₄-Monohydroxyalkylgruppe, C₂₋₄-Polyhydroxyalkylgruppe, C₁₋₄-Aminoalkylgruppe, C₁₋₄-Alkoxy-C₁₋₄-Alkylgruppe, C₁₋₄-Acetylaminoalkylgruppe, C₁₋₄-Alkyl-C₁₋₄-Aminoalkylgruppe, C₁₋₄-Dialkyl-C₁₋₄-Aminoalkylgruppe, C₁₋₄-Trialkyl-C₁₋₄-Ammonioalkylgruppe eines C₁₋₄-Alkylhalogenids oder eines C₁₋₄-Alkylsulfats, eine gegebenenfalls substituierte Benzylgruppe, eine gegebenenfalls substituierte Phenylgruppe, C₁₋₄-Sulfoalkylgruppe und C₁₋₄-Alkoxy-C₁₋₄-Carbonylalkyl,
und die kosmetisch akzeptablen Salze dieser Verbindungen.

2. Verbindung nach Anspruch 1, dadurch gekennzeichnet, daß die Gruppen Alkyl, Hydroxyalkyl oder Alkoxyalkyl die Gruppen Methyl, Ethyl, Propyl, Butyl, Hydroxyethyl, Hydroxypropyl, Dihydroxypropyl oder Ethoxyethyl bedeuten.

3. Verbindungen nach Anspruch 1 oder 2, dadurch gekennzeichnet, daß die Gruppe R₁ Wasserstoff bedeutet.

4. Verbindungen nach einem der Ansprüche 1 bis 3, dadurch gekennzeichnet, daß sie unter den folgenden Verbindungen ausgewählt sind:
- 2-Nitro-5-trimethylammonioethylthio-p-phenylendiamin,
- 2-Nitro-4-N-(β-hydroxyethyl)amino-5-[(dimethyl)aminoethyl]thio-p-anilin,
- 2-Nitro-5-(β-aminoethyl)thio-p-phenylendiamin,
- 2-Nitro-4-N -(β,γ-dihydroxypropyl)amino-5-methylthioanilin,
- 2-Nitro-5-methyltio-p-phenylendiamin,
- 2-Nitro-5-ethylthio-p-phenylendiamin,
- 2-Nitro-5-butylthio-p-phenylendiamin,
- 2-Nitro-5-β-hydroxyethylthio-p-phenylendiamin,
- 2-Nitro-5-(β,γ-dihydroxypropyl)thio-p-phenylendiamin,
- 1,4-N-di-(β-hydroxyethyl)amino-2-nitro-5-ethoxyethylthiobenzol,
- 2-Nitro-4-N-(β-hydroxyethyl)amino-5-β-hydroxyethylthioanilin,
- 2-Nitro-5-(p-methoxybenzyl)thio-p-phenylendiamin,
- 2-Nitro-4-N-(β-aminoethyl)amino-5-methylthioanilin,
- 2-Nitro-4-N-(diethylaminoethyl)amino-5-methylthioanilin,
- 2-Nitro-4-N-(diethylaminoethyl)amino-5-ethylthioanilin,
- 2-Nitro-4-N-(β,γ-dihydroxypropyl)amino-5-acetylaminoethylthioanilin,
- 2-Nitro-5-acetylaminoethylthio-p-phenylendiamin,
- 2-Nitro-5-[(dimethyl)aminoethyl]thio-p-phenylendiamin,
- 2-Nitro-5-(β-aminoethyl)thio-p-phenylendiamin,
- 2-Nitro-4-amino-5-(β-hydroxypropyl)thio-N-(methyl)anilin,p
- 2-Nitro-4-amino-5-(p-hydroxyphenyl)thio-N-(methyl)anilin,
- 2-Nitro-5-ethoxycarbonylethyltio-p-phenylendiamin,
- 2-Nitro-5-methylsulfinyl-p-phenylendiamin,
- 2-Nitro-5-mesyl-p-phenylendiamin,
- 1-N-(β-hydroxyethyl)amino-2-nitro-4,N,N-bis-(β-hydroxyethyl)amino-5-isopropylthiobenzol,
- 2-Nitro-5-sulfoethylthio-p-phenylendiamin,
und die kosmetisch akzeptablen Salze dieser Verbindungen.

5. Verfahren zur Herstellung einer Verbindung der Formel (I) nach einem der Ansprüche 1 bis 4, dadurch gekennzeichnet, daß es darin besteht, in einem ersten Schritt in einem Lösungsmittelmedium bei einer Temperatur im Bereich von Raumtemperatur bis zur Rückflußtemperatur des Reaktionsgemisches in Gegenwart eines Mittels zum Abfangen der Halogenwasserstoffsäure eine Verbindung der Formel (II): 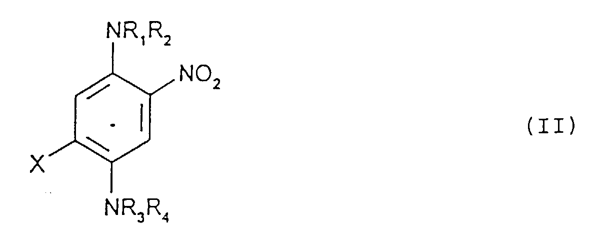 worin R₁, R₂, R₃ und R₄ die in Anspruch 1 angegebenen Bedeutungen aufweisen und X ein Halogenatom bedeutet, das unter Chlor, Brom und Iod ausgewählt ist,
mit einem Thiol der Formel (III)
ASZ (III)
umzusetzen, worin Z die in Anspruch 1 angegebenen Bedeutungen aufweist und A ein Wasserstoffatom, Natriumatom oder Kaliumatom bedeutet, um eine Verbindung der Formel (I), worin n Null ist, herzustellen. 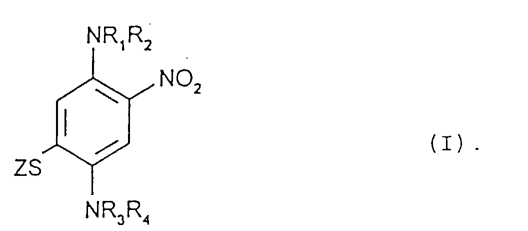 In einem zweiten Schritt wird die Verbindung der Formel (I), worin n Null ist, gegebenenfalls in einem Lösungsmittelmedium in Gegenwart eines Oxidationsmittels für Schwefel, wie Kaliummonopersulfat, bei einer Temperatur im Bereich von 0 bis 40 °C oxidiert, um eine Verbindung der Formel (I) herzustellen, worin n = 1 oder 2 ist.

6. Herstellungsverfahren nach Anspruch 5, dadurch gekennzeichnet, daß das Lösungsmittel unter 1,2-diMethoxyethan, Dimethylformamid, N-Methylpyrrolidon und Dioxan ausgewählt ist.

7. Herstellungsverfahren nach Anspruch 5 oder 6, dadurch gekennzeichnet, daß das Mittel zum Abfangen der Halogenwasserstoffsäure unter Natriumhydroxid, Kaliumhydroxid, Triethylamin, Natriumcarbonat, Kaliumcarbonat und Calciumcarbonat und den verwendeten Thiolsalzen der Formel (III) ausgewählt ist.

8. Färbemittelzusammensetzung zum direkten Färben von Keratinfasern und insbesondere von menschlichen Keratinfasern, dadurch gekennzeichnet, daß sie in einem wässerigen, alkoholischen oder wässerig-alkoholischen Träger mindestens eine Verbindung der Formel (I): 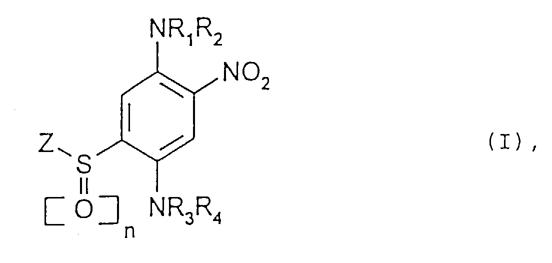 worin bedeuten:
- n = 0, 1 oder 2;
- R₁, R₂, R₃ oder R₄, die gleich oder voneinander verschieden sind, ein Wasserstoffatom, eine geradkettige oder verzweigte C₁₋₄-Alkylgruppe, eine C₁₋₄-Monohydroxyalkylgruppe, eine C₂₋₄-Polyhydroxyalkylgruppe, eine C₁₋₄-Aminoalkylgruppe und eine Mono- oder Dialkylaminoalkylgruppe, wobei die Alkylgruppen 1 bis 4 Kohlenstoffatome aufweisen;
- Z eine geradkettige oder verzweigte C₁₋₄-Alkylgruppe, C₁₋₄-Monohydroxyalkylgruppe, C₂₋₄-Polyhydroxyalkylgruppe, C₁₋₄-Aminoalkylgruppe, C₁₋₄-Alkoxy-C₁₋₄-Alkylgruppe, C₁₋₄-Acetylaminoalkylgruppe, C₁₋₄-Alkyl-C₁₋₄-Aminoalkylgruppe, C₁₋₄-Dialkyl-C₁₋₄-Aminoalkylgruppe, C₁₋₄-Trialkyl-C₁₋₄-Ammonioalkylgruppe eines C₁₋₄-Alkylhalogenids oder eines C₁₋₄-Alkylsulfats, eine gegebenenfalls substituierte Benzylgruppe, eine gegebenenfalls substituierte Phenylgruppe, C₁₋₄-Sulfoalkylgruppe und C₁₋₄-Alkoxy-C₁₋₄-Carbonylalkylgruppe;
oder eines der kosmetisch akzeptablen Salze dieser Verbindungen enthält.

9. Färbemittelzusammensetzung nach Anspruch 8, dadurch gekennzeichnet, daß die Gruppen Alkyl, Hydroxyalkyl oder Alkoxy der Verbindungen der Formel (I) die Gruppen Methyl, Ethyl, Propyl, Butyl, Hydroxyethyl, Hydroxypropyl, Dihydroxypropyl oder Ethoxyethyl bedeuten.

10. Färbemittelzusammensetzung nach Anspruch 8 oder 9, dadurch gekennzeichnet, daß die Gruppe R₁ der Verbindungen der Formel (I) ein Wasserstoffatom bedeutet.

11. Färbemittelzusammensetzung nach einem der Ansprüche 8 bis 10, dadurch gekennzeichnet, daß sie eine Verbindung der Formel (I) enthält, die ausgewählt ist unter:
- 2-Nitro-5-trimethylammonioethylthio-p-phenylendiamin,
- 2-Nitro-4-N-(β-hydroxyethyl)amino-5-[(dimethyl)aminoethyl]thio-anilin,
- 2-Nitro-5-(β-aminoethyl)thio-p-phenylendiamin,
- 2-Nitro-4-N-(β,γ-dihydroxypropyl)amino-5-methylthioanilin,
- 2-Nitro-5-methyltio-p-phenylendiamin,
- 2-Nitro-5-ethylthio-p-phenylendiamin,
- 2-Nitro-5-butylthio-p-phenylendiamin,
- 2-Nitro-5-β-hydroxyethylthio-p-phenylendiamin,
- 2-Nitro-5-(β,γ-dihydroxypropyl)thio-p-phenylendiamin,
- 1,4-N-di-(β-hydroxyethyl)amino-2-nitro-5-ethoxyethylthiobenzol,
- 2-Nitro-4-N-(β-hydroxyethyl)amino-5-β-hydroxyethylthioanilin,
- 2-Nitro-5-(p-methoxybenzyl)thio-p-phenylendiamin,
- 2-Nitro-4-N-(β-aminoethyl)amino-5-methylthioanilin,
- 2-Nitro-4-N-(diethylaminoethyl)amino-5-methylthioanilin,
- 2-Nitro-4-N-(diethylaminoethyl)amino-5-ethylthioanilin,
- 2-Nitro-4-N-(β,γ-dihydroxypropyl)amino-5-acetylaminoethylthioanilin,
- 2-Nitro-5-acetylaminoethylthio-p-phenylendiamin,
- 2-Nitro-5-[(dimethyl)aminoethyl]thio-p-phenylendiamin,
- 2-Nitro-5-(β-aminoethyl)thio-p-phenylendiamin,
- 2-Nitro-4-amino-5-(β-hydroxypropyl)thio-N-(methyl)anilin,
- 2-Nitro-4-amino-5-(p-hydroxyphenyl)thio-N-(methyl)anilin,
- 2-Nitro-5-ethoxycarbonylethyltio-p-phenylendiamin,
- 2-Nitro-5-methylsulfinyl-p-phenylendiamin,
- 2-Nitro-5-mesyl-p-phenylendiamin,
- 1-N-β-hydroxyethyl)amino-2-nitro-4,N,N-bis-(β-hydroxyethyl)amino-5-isopropylthiobenzol,
- 2-Nitro-5-sulfoethylthio-p-phenylendiamin,
sowie die kosmetisch akzeptablen Salze dieser Verbindungen.

12. Färbemittelzusammensetzung nach einem der Ansprüche 8 bis 11, dadurch gekennzeichnet, daß sie mindestens ein gelbes oder gelb-grünes nitriertes Benzolfärbemittel enthält, das an grauen Haaren mit 90 % weißen Haaren eine Farbnuance im Bereich von 2,4 Y bis 0,2 YR nach der MUNSELL-Bezeichnung ergibt.

13. Färbemittelzusammensetzung nach Anspruch 12, dadurch gekennzeichnet, daß die gelben oder gelb-grünen nitrierten Benzolfärbemittel unter den folgenden Verbindungen ausgewählt sind:
- 1-β-Hydroxyethyloxy-3-methylamino-4-nitrobenzol,
- 1-(Methylamino)-2-nitro-5(β,γ-dihydroxypropyl)oxybenzol,
- 1-(β-Hydroxyethylamino)-2-methoxy-4-nitrobenzol,
- 1-(β-Aminoethylamino)-2-nitro-5-methoxybenzol,
- 1,3-Di-(β-Hydroxyethylamino)-4-nitro-6-chlorbenzol,
- 1-Amino-2-nitro-6-methylbenzol,
- 1-(β-Hydroxyethylamino)-2-hydroxy-4-nitrobenzol,
- N-(β-Hydroxyethyl)-2-nitro-4-trifluormethylanilin,
- 4-β-Hydroxyethylamino-3-nitro-benzolsulfonsäure,
- 4-Ethylamino-3-nitro-benzoesäure,
- 4-(β-Hydroxyethyl)amino-3-nitro-chlorbenzol,
- 4-(β-Hydroxyethyl)amino-3-nitro-methylbenzol,
- 4-(β,γ-Dihydroxypropyl)amino-3-nitro-trifluormethylbenzol,
- 1-β-Ureidoethylamino-4-nitrobenzol,
- O,N-bis(β-Hydroxyethyl)-2-amino-5-nitrophenol,
- 1,3-Diamino-4-nitrobenzol,
- 1-Hydroxy-2-amino-5-nitrobenzol,
- 1-Amino-2-[tris(hydroxymethyl)methyl]amino-5-nitrobenzol,
- 1-(β-Hydroxyethyl)amino-2-nitrobenzol,
- 4-(β-Hydroxyethylamino)-3-nitrobenzamid.

14. Färbemittelzusammensetzung nach einem der Ansprüche 8 bis 13, dadurch gekennzeichnet, daß sie mindestens ein blaues nitriertes Benzolfärbemittel enthält, das unter den folgenden Verbindungen ausgewählt ist:
- 1-(β-Hydroxyethyl)amino-4-N,N-bis(β-hydroxyethyl)amino-2-nitrobenzol,
- 1-(y-Hydroxypropyl)amino-4-N,N-bis(β-hydroxyethyl)amino-2-nitrobenzol,
- 1-(β-Hydroxyethyl)amino-4-(N-methyl-N-β-hydroxyethyl)amino-2-nitrobenzol,
- 1-(β-Hydroxyethyl)amino-4-(N-ethyl-N-β-hydroxyethyl)amino-2-nitrobenzol,
- 1-(β,γ-Dihydroxypropyl)amino-4-(N-ethyl-N-β-hydroxyethyl)amino-2-nitrobenzol,
- 2-Nitro-p-phenylendiamine der Formel: 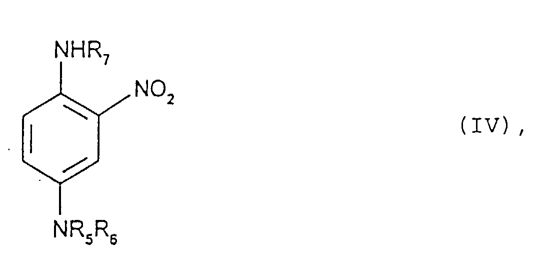 worin bedeuten:
- R₅ C₁₋₄-Alkyl, β-Hydroxyethyl, β-Hydroxypropyl oder γ-Hydroxypropyl;
- R₆ und R₇ unabhängig voneinander β-Hydroxyethyl, β-Hydroxypropyl, γ-Hydroxypropyl oder β,y-Dihydroxypropyl, wobei mindestens eine der Gruppen R₅, R₆ oder R₇ eine γ-Hydroxypropylgruppe bedeutet und R₅ und R₆ nicht gleichzeitig eine β-Hydroxyethylgruppe bedeuten können, wenn R₇ eine γ-Hydroxypropylgruppe ist, wie dies in der Patentanmeldung FR 92-07515 beschrieben ist.

15. Färbemittelzusammensetzung nach einem der Ansprüche 8 bis 14, dadurch gekennzeichnet, daß sie mindestens ein rotes nitriertes Benzolfärbemittel enthält, das unter den folgenden Verbindungen ausgewählt ist:
- 1-Hydroxy-3-nitro-4-(γ-hydroxypropylamino) benzol,
- N-(β-Hydroxyethyl)amino-3-nitro-4-aminobenzol,
- 1-Amino-3-methyl-4-(β-hydroxyethyl)amino-6-nitrobenzol,
- 1-Hydroxy-3-nitro-4-N-β-hydroxyethyl-aminobenzol,
- 1,4-Diamino-2-nitrobenzol,
- 1-Amino-2-nitro-4-methylaminobenzol,
- N-(β-Hydroxyethyl)-2-nitro-p-phenylendiamin,
- 1-Amino-2-nitro-4-(β-hydroxyethylamino)-5-chlorbenzol,
- 2-Nitro-4-amino-diphenylamin,
- 1-Amino-3-nitro-6-hydroxybenzol.

16. Färbemittelzusammensetzung nach einem der Ansprüche 8 bis 15, dadurch gekennzeichnet, daß sie mindestens ein oranges nitriertes Benzolfärbemittel enthält, das unter den folgenden Verbindungen ausgewählt ist:
- 1-(β-Aminoethyl)amino-2-nitro-4-(β-hydroxyethyl)oxybenzol,
- 1-(β,γ-dihydroxypropyl)oxy-3-nitro-4-(β-hydroxyethyl)aminobenzol,
- 1-Hydroxy-3-nitro-4-aminobenzol,
- 1-Hydroxy-2-amino-4,6-dinitrobenzol,
- 1-Methoxy-3-nitro-4-(β-hydroxyethylamino)benzol,
- 2-Nitro-4'-hydroxydiphenylamin,
- 1-Amino-2-nitro-4-hydroxy-5-methylbenzol.

17. Färbemittelzusammensetzung nach einem der Ansprüche 8 bis 16, dadurch gekennzeichnet, daß sie weitere Direktfarbstoffe enthält, die unter den Azofarbstoffen, Anthrachinonfarbstoffen, den Triarylmethanderivaten und den basischen Färbemitteln ausgewählt sind.

18. Färbemittelzusammensetzung nach einem der Ansprüche 8 bis 17, dadurch gekennzeichnet, daß sie die Verbindung der Formel (I) in Mengenanteilen im Bereich von 0,01 bis 10 Gew.-%, ausgedrückt als freie Base und bezogen auf das Gesamtgewicht der Zusammensetzung, enthält.

19. Färbemittelzusammensetzung nach einem der Ansprüche 12 bis 18, dadurch gekennzeichnet, daß sie ferner 0,05 bis 3 Gew.% der gelben und/oder gelb-grünen und/oder blauen nitrierten Benzolfärbemittel, bezogen auf das Gesamtgewicht der Zusammensetzung, enthält.

20. Färbemittelzusammensetzung nach einem der Ansprüche 15 bis 19, dadurch gekennzeichnet, daß sie ferner 0,05 bis 10 Gew.-% weitere Direktfarbstoffe enthält.

21. Färbemittelzusammensetzung nach einem der Ansprüche 8 bis 20, dadurch gekennzeichnet, daß sie mindestens ein organisches Lösungsmittel enthält, das unter den Alkoholen, Glykolen und Glykolethern in Mengenanteilen im Bereich von 0,5 bis 20 Gew.-%, bezogen auf das Gesamtgewicht der Zusammensetzung, ausgewählt ist.

22. Färbemittelzusammensetzung nach einem der Ansprüche 8 bis 21, dadurch gekennzeichnet, daß sie mindestens einen Hilfsstoff enthält, der unter den Fettamiden, anionischen, kationischen, nichtionischen, amphoteren, zwitterionischen grenzflächenaktiven Stoffen und deren Gemischen, Verdickungsmitteln, Antioxidantien, Parfums, Maskierungsmitteln, Filmbildnern, Haarbehandlungsmitteln, Dispergiermitteln, Haarkonditioniermitteln, Konservierungsmitteln und Trübungsmitteln ausgewählt ist.

23. Färbemittelzusammensetzung nach einem der Ansprüche 8 bis 22, dadurch gekennzeichnet, daß sie einen pH-Wert im Bereich von 4 bis 11 aufweist.

24. Verfahren zum Färben von Keratinfasern und insbesondere von menschlichen Keratinfasern durch Direktfärbung, dadurch gekennzeichnet, daß die Färbemittelzusammensetzung nach einem der Ansprüche 8 bis 23 auf die trockenen oder feuchten Keratinfasern aufgebracht wird, und die Keratinfasern ohne zwischenzeitliches Spülen getrocknet werden.

25. Verfahren zum Färben von Keratinfasern und insbesondere von menschlichen Keratinfasern durch Direktfärbung, dadurch gekennzeichnet, daß die Färbemittelzusammensetzung nach einem der Ansprüche 8 bis 23 auf die trockenen oder feuchten Keratinfasern aufgebracht wird, und daß die Zusammensetzung 3 bis 60 min und vorzugsweise 5 bis 45 min einwirken kann, die Keratinfasern gespült und gegebenenfalls gewaschen und von neuem gespült und anschließend getrocknet werden.

## Claims

1. 2-Nitro-para-phenylenediamine substituted with sulphur in the 5-position, of formula: 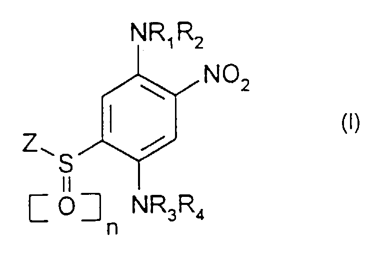 in which:
- n = 0, 1 or 2;
- R₁, R₂, R₃ and R₄, which are identical or different, are a hydrogen atom or a linear or branched C₁-C₄-alkyl, C₁-C₄-monohydroxyalkyl, C₂-C₄-polyhydroxyalkyl, C₁-C₄-aminoalkyl or C₁-C₄-monoalkyl- or -dialkyl-amino(C₁-C₄)-alkyl radical; and
- Z is a linear or branched C₁-C₄-alkyl, C₁-C₄-monohydroxyalkyl, C₂-C₄-polyhydroxyalkyl, C₁-C₄-aminoalkyl, C₁-C₄-alkoxy(C₁-C₄)alkyl, acetylamino(C₁-C₄)alkyl, C₁-C₄-alkylamino(C₁-C₄)alkyl or C₁-C₄-dialkylamino(C₁-C₄)alkyl radical, a C₁-C₄-trialkylammonio(C₁-C₄)alkyl radical of a C₁-C₄-alkyl halide or of a C₁-C₄-alkyl sulphate, or an optionally substituted benzyl, optionally substituted phenyl, C₁-C₄-sulphoalkyl or C₁-C₄-alkoxycarbonyl(C₁-C₄)alkyl radical,
and the cosmetically acceptable salts of these compounds.

2. Compound according to Claim 1, characterized in that the alkyl, hydroxyalkyl or alkoxyalkyl radicals are methyl, ethyl, propyl, butyl, hydroxyethyl, hydroxypropyl, dihydroxypropyl or ethoxyethyl.

3. Compound according to Claim 1 or 2, characterized in that the radical R₁ is a hydrogen atom.

4. Compound according to any one of Claims 1 to 3, characterized in that it is selected from the following compounds:
- 2-nitro-5-trimethylammonioethylthio-para-phenylene-diamine,
- 2-nitro-4-N-(β-hydroxyethyl)amino-5-[(dimethyl)aminoethyl]thio aniline
- 2-nitro-5-(β-aminoethyl)thio-para-phenylenediamine,
- 2-nitro-4-N-(β,γ-dihydroxypropyl)amino-5-methylthioaniline,
- 2-nitro-5-methylthio-para-phenylenediamine,
- 2-nitro-5-ethylthio-para-phenylenediamine,
- 2-nitro-5-butylthio-para-phenylenediamine,
- 2-nitro-5-β-hydroxyethylthio-para-phenylenediamine,
- 2-nitro-5-β,γ-dihydroxypropyl)thio-para-phenylenediamine,
- 1,4-N-di(β-hydroxyethyl)amino-2-nitro-5-ethoxyethylthiobenzene,
- 2-nitro-4-N-(β-hydroxyethyl)amino-5-β-hydroxyethylthioaniline,
- 2-nitro-5-(paramethoxybenzyl)thio-para-phenylenediamine,
- 2-nitro-4-N-(β-aminoethyl)amino-5-methylthioaniline,
- 2-nitro-4-N-(diethylaminoethyl)amino-5-methylthioaniline,
- 2-nitro-4-N-(diethylaminoethyl)amino-5-ethylthioaniline,
- 2-nitro-4-N-β,γ-dihydroxypropyl)amino-5-acetylaminoethylthioaniline,
- 2-nitro-5-acetylaminoethylthio-para-phenylenediamine,
- 2-nitro-5-[(dimethyl)aminoethyl]thio-para-phenylenediamine,
- 2-nitro-5-(β-aminoethyl)thio-para-phenylenediamine,
- 2-nitro-4-amino-5-(β-hydroxypropyl)thio-N-(methyl)aniline,
- 2-nitro-4-amino-5-(parahydroxyphenyl)thio-N-(methyl)aniline,
- 2-nitro-5-ethoxycarbonylethylthio-para-phenylenediamine,
- 2-nitro-5-methylsulphinyl-para-phenylenediamine,
- 2-nitro-5-mesyl-para-phenylenediamine,
- 1-N-(β-hydroxyethyl)amino-2-nitro-4-N,N-bis(β-hydroxyethyl)amino-5-isopropylthiobenzene,
- 2-nitro-5-sulphoethylthio-para-phenylenediamine
and the cosmetically acceptable salts of these compounds.

5. Process for the preparation of a compound of formula (I) according to any one of Claims 1 to 4, characterized in that it consists, in a first step, in reacting a compound of formula (II): 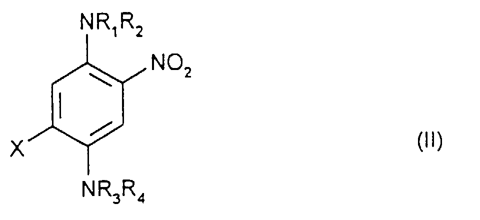 in which R₁, R₂, R₃ and R₄ are defined as indicated in Claim 1 and X is a halogen atom selected from chlorine, bromine and iodine, with a thiol of formula (III):
ASZ (III)
in which Z is defined as indicated in Claim 1 and A is a hydrogen, sodium or potassium atom, in a solvent medium, at a temperature between room temperature and the reflux temperature of the reaction medium, in the presence of a hydrohalic acid acceptor, to give a compound of formula (I) in which n = 0: 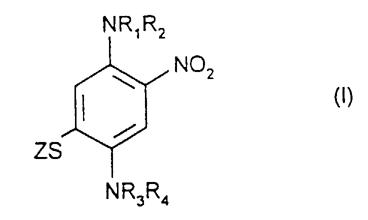 In a second step, the compound of the formula (I) in which n = 0 is optionally oxidized in a solvent medium, in the presence of an oxidizing agent for sulphur, such as monopotassium persulphate, at a temperature of between zero and 40°C, to give a compound of formula (I) in which n = 1 or 2.

6. Preparative process according to Claim 5, characterized in that the solvent is selected from 1,2-dimethoxyethane, dimethylformamide, N-methylpyrrolidone and dioxane.

7. Preparative process according to Claim 5 or 6, characterized in that the hydrohalic acid acceptor is selected from sodium hydroxide, potassium -hydroxide, triethylamine, sodium, potassium and calcium carbonates and the salts of the thiol of formula (III) which are used.

8. Dye composition for the direct dyeing of keratin fibres and in particular human hair, characterized in that it contains, in an aqueous, alcoholic or aqueous-alcoholic vehicle, at least one compound of formula (I): 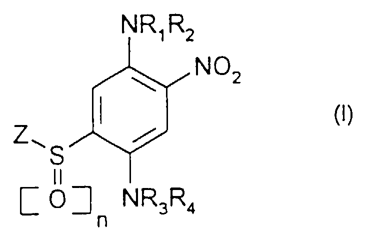 in which:
- n = 0, 1 or 2;
- R₁, R₂, R₃ and R₄, which are identical or different, are a hydrogen atom or a linear or branched C₁-C₄-alkyl, C₁-C₄-monohydroxyalkyl, C₂-C₄-polyhydroxyalkyl, C₁-C₄-aminoalkyl or C₁-C₄-monoamkyo- or -dialkyl-amino(C₁-C₄)-alkyl radical; and
- Z is a linear or branched C₁-C₄-aSky-, C₁-C₄-mono-hydroxyalkyl, C₂-C₄-polyhydroxyalkyl, C₁-C₄-aminoalkyl, C₁-C₄-alkoxy(C₁-C₄)alkyl, acetylamino(C₁-C₄)alkyl, C₁-C₄-alkylamino(C₁-C₄)alkyl or C₁-C₄-dialkylamino(C₁-C₄)alkyl radical, a C₁-C₄-trialkylammonio(C₁-C₄)alkyl radical of a C₁-C₄-alkyl halide or of a C₁-C₄-alkyl sulphate, or an optionally substituted benzyl, optionally substituted phenyl, C₁-C₄-sulphoalkyl or C₁-C₄-alkoxycarbonyl-(C₁-C₄)alkyl radical,
or one of its cosmetically acceptable salts.

9. Dye composition according to Claim 8, characterized in that the alkyl, hydroxyalkyl or alkoxyalkyl radicals of the compound of formula (I) are methyl, ethyl, propyl, butyl, hydroxyethyl, hydroxypropyl, dihydroxypropyl or ethoxyethyl.

10. Dye composition according to Claim 8 or 9, characterized in that the radical R₁ of the compound of formula (I) is a hydrogen atom.

11. Dye composition according to any one of Claims 8 to 10, characterized in that it contains a compound of formula (I) selected from:
- 2-nitro-5-trimethylammonioethylthio-para-phenylene-diamine,
- 2-nitro-4-N-(β-hydroxyethyl)amino-5-[(dimethyl)aminoethyl] thio-aniline
- 2-nitro-5-(β-aminoethyl)thio-para-phenylenediamine,
- 2-nitro-4-N-(β,γ-dihydroxypropyl)amino-5-methylthioaniline,
- 2-nitro-5-methylthio-para-phenylenediamine,
- 2-nitro-5-ethylthio-para-phenylenediamine,
- 2-nitro-5-butylthio-para-phenylenediamine,
- 2-nitro-5-β-hydroxyethylthio-para-phenylenediamine,
- 2-nitro-5-(β,γ-dihydroxypropyl)thio-para-phenylene-diamine,
- 1,4-N-di(β-hydroxyethyl)amino-2-nitro-5-ethoxyethylthiobenzene,
- 2-nitro-4-N-(β-hydroxyethyl)amino-5-β-hydroxyethylthioaniline,
- 2-nitro-5-(paramethoxybenzyl)thio-para-phenylenediamine,
- 2-nitro-4-N-(β-aminoethyl)amino-5-methylthioaniline,
- 2-nitro-4-N-(diethylaminoethyl)amino-5-methylthioaniline,
- 2-nitro-4-N-(diethylaminoethyl)amino-5-ethylthioaniline,
- 2-nitro-4-N-(β,γ-dihydroxypropyl)amino-5-acetylaminoethylthioaniline,
- 2-nitro-5-acetylaminoethylthio-para-phenylenediamine,
- 2-nitro-5-[(dimethyl)aminoethyl]thio-para-phenylenediamine,
- 2-nitro-5-(β-aminoethyl)thio-para-phenylenediamine,
- 2-nitro-4-amino-5-(β-hydroxypropyl)thio-N-(methyl)aniline,
- 2-nitro-4-amino-5-(parahydroxyphenyl)thio-N-(methyl)aniline,
- 2-nitro-5-ethoxycarbonylethylthio-para-phenylenediamine,
- 2-nitro-5-methylsulphinyl-para-phenylenediamine,
- 2-nitro-5-mesyl-para-phenylenediamine,
- 1-N-(β-hydroxyethyl)amino-2-nitro-4-N,N-bis(β-hydroxyethyl)amino-5-isopropylthiobenzene,
- 2-nitro-5-sulphoethylthio-para-phenylenediamine
and the cosmetically acceptable salts of these compounds.

12. Dye composition according to any one of Claims 8 to 11, characterized in that it contains at least one yellow or yellow-green nitrobenzene dye with a Munsell shade of between 2.4 Y and 0.2 YR on grey hair containing 90% white hairs.

13. Dye composition according to Claim 12, characterized in that the yellow or yellow-green nitrobenzene dye is selected from the following compounds:
- 1-β-hydroxyethoxy-3-methylamino-4-nitrobenzene,
- 1-(methylamino)-2-nitro-5-(β,γ-dihydroxypropyl)oxybenzene,
- 1-(β-hydroxyethylamino)-2-methoxy-4-nitrobenzene,
- 1-(β-aminoethylamino)-2-nitro-5-methoxybenzene,
- 1,3-di(β-hydroxyethylamino)-4-nitro-6-chlorobenzene,
- 1-amino-2-nitro-6-methylbenzene,
- 1-(β-hydroxyethylamino)-2-hydroxy-4-nitrobenzene,
- N-(β-hydroxyethyl)-2-nitro-4-trifluoromethylaniline,
- 4-β-hydroxyethylamino-3-nitrobenzenesulphonic acid,
- 4-ethylamino-3-nitrobenzoic acid,
- 4-(β-hydroxyethyl)amino-3-nitrochlorobenzene,
- 4-(β-hydroxyethyl)amino-3-nitromethylbenzene,
- 4-(β,γ-dihydroxypropyl)amino-3-nitrotrifluoromethylbenzene,
- 1-β-ureidoethylamino-4-nitrobenzene,
- O,N-bis(β-hydroxyethyl)-2-amino-5-nitrophenol,
- 1,3-diamino-4-nitrobenzene,
- 1-hydroxy-2-amino-5-nitrobenzene,
- 1-amino-2-[tris(hydroxymethyl)methyl]amino-5-nitrobenzene,
- 1-(β-hydroxyethyl)amino-2-nitrobenzene and
- 4-(β-hydroxyethylamino)-3-nitrobenzamide.

14. Dye composition according to any one of Claims 8 to 13, characterized in that it contains at least one blue nitrobenzene dye selected from the following compounds:
- 1-(β-hydroxyethyl)amino-4-N,N-bis(β-hydroxyethyl)amino-2-nitrobenzene,
- 1-(γ-hydroxypropyl)amino-4-N,N-bis(β-hydroxyethyl)amino-2-nitrobenzene,
- 1-(β-hydroxyethyl)amino-4-(N-methyl,N-β-hydroxyethyl)amino-2-nitrobenzene,
- 1-(β-hydroxyethyl)amino-4-(N-ethyl,N-β-hydroxyethyl)amino-2-nitrobenzene,
- 1-(β,γ-dihydroxypropyl)amino-4-(N-ethyl,N-β-hydroxyethyl)amino-2-nitrobenzene and
- the 2-nitro-para-phenylenediamines of formula: 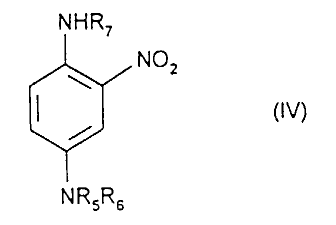 in which:
- R₅ is a C₁-C₄-alkyl, β-hydroxyethyl, β-hydroxypropyl or γ-hydroxypropyl radical; and
- R₆ and R₇ independently of one another are a β-hydroxy-ethyl, β-hydroxypropyl, γ-hydroxypropyl or β,γ-dihydroxypropyl radical, at least one of the radicals R₅, R₆ or R₇ being a γ-hydroxypropyl radical and it being impossible for R₅ and R₆ simultaneously to be a β-hydroxyethyl radical when R₇ is a γ-hydroxypropyl radical,
as described in patent application FR 92/07515.

15. Dye composition according to any one of Claims 8 to 14, characterized in that it contains at least one red nitrobenzene dye selected from the following compounds:
- 1-hydroxy-3-nitro-4-(y-hydroxypropylamino)benzene,
- N-(β-hydroxyethyl)amino-3-nitro-4-aminobenzene,
- 1-amino-3-methyl-4-(β-hydroxyethyl)amino-6-nitrobenzene,
- 1-hydroxy-3-nitro-4-N-β-hydroxyethylaminobenzene,
- 1,4-diamino-2-nitrobenzene,
- 1-amino-2-nitro-4-methylaminobenzene,
- N-(β-hydroxyethyl)-2-nitro-para-phenylenediamine,
- 1-amino-2-nitro-4-(β-hydroxyethylamino)-5-chlorobenzene,
- 2-nitro-4-aminodiphenylamine and
- 1-amino-3-nitro-6-hydroxybenzene.

16. Dye composition according to any one of Claims 8 to 15, characterized in that it contains at least one orange nitrobenzene dye selected from the following compounds:
- 1-(β-aminoethyl)amino-2-nitro-4-(β-hydroxyethyl)oxybenzene,
- 1-(β,γ-dihydroxypropyl)oxy-3-nitro-4-(β-hydroxyethyl)aminobenzene,
- 1-hydroxy-3-nitro-4-aminobenzene,
- 1-hydroxy-2-amino-4,6-dinitrobenzene,
- 1-methoxy-3-nitro-4 -(β-hydroxyethylamino)benzene,
- 2-nitro-4'-hydroxydiphenylamine and
- 1-amino-2-nitro-4-hydroxy-5-methylbenzene.

17. Dye composition according to any one of Claims 8 to 16, characterized in that it contains other direct dyes selected from azo dyes, anthraquinone dyes, triarylmethane derivatives and basic dyes.

18. Dye composition according to any one of Claims 8 to 17, characterized in that it contains the compound of formula (I) in proportions, expressed as the free base, of between 0.01 and 10 % by weight, relative to the total weight of the composition.

19. Dye composition according to any one of Claims 12 to 18, characterized in that it also contains from 0.05 to 3 % by weight of yellow and/or yellow-green and/or blue nitrobenzene dyes, relative to the total weight of the composition.

20. Dye composition according to any one of Claims 15 to 19, characterized in that it also contains 0.05 to 10 % by weight of other direct dyes.

21. Dye composition according to any one of Claims 8 to 20, characterized in that it contains at least one organic solvent selected from alcohols, glycols and glycol ethers, in proportions of between 0.5 % and 20 %, relative to the total weight of the composition.

22. Dye composition according to any one of Claims 8 to 21, characterized in that it contains at least one adjuvant selected from fatty amides, anionic, cationic, nonionic, amphoteric and zwitterionic surfactants and mixtures thereof, thickeners, antioxidants, fragrances, sequestering agents, film-forming agents, hair treating agents, dispersing agents, hair conditioners, preserving agents and opacifiers.

23. Dye composition according to any one of Claims 8 to 22, characterized in that it has a pH of between 4 and 11.

24. Method of dyeing keratin fibres, and in particular human hair, by direct dyeing, characterized in that the dye composition according to any one of Claims 8 to 23 is applied to the dry or wet keratin fibres and these keratin fibres are dried without intermediate rinsing.

25. Method of dyeing keratin fibres, and in particular human hair, by direct dyeing, characterized in that the dye composition according to any one of Claims 8 to 23 is applied to the dry or wet keratin fibres and in that, after the composition has been allowed to act for 3 to 60 minutes, preferably for 5 to 45 minutes, the keratin fibres are rinsed, optionally washed and rinsed again, and then dried.
